# EUROPEAN PATENT SPECIFICATION

(11) **EP 3 187 515 B1**
(45) Date of publication and mention of the grant of the patent: **18.03.2020**
(21) Application number: 15836220.2
(22) Date of filing: 25.08.2015
(51) Int. Cl.: C07C 69/76, C07C 69/82, C08F 20/30

(54) **POLYMERIZABLE MONOMER, CURABLE COMPOSITION AND RESIN MEMBER**
POLYMERISIERBARES MONOMER, HÄRTBARE ZUSAMMENSETZUNG UND HARZELEMENT
MONOMÈRE POLYMÉRISABLE, COMPOSITION DURCISSABLE ET ÉLÉMENT EN RÉSINE

(30) Priority: 26.08.2014 JP 2014171512
(43) Date of publication of application: 05.07.2017
(73) Proprietor: Tokuyama Dental Corporation, Tokyo 110-0016 (JP)
(72) Inventor: INAKI, Yoshitaka, Tokyo 110-0016 (JP); SAKATA, Eibu, Tokyo 110-0016 (JP); SUZUKI, Takeshi, Tokyo 110-0016 (JP)
(74) Representative: Gill Jennings & Every LLP
(86) International application number: PCT/JP2015/073901
(87) International publication number: WO 2016/031829

(56) References cited:
- WO-A1-2014/172138
- JP-A- H05 170 705
- JP-A- H05 222 127
- JP-A- H08 184 961
- JP-A- S59 232 849
- JP-A- S62 240 647
- US-A1- 2004 176 496

## Description

### Technical Field

The present invention relates to a polymerizable monomer, a method of producing the polymerisable monomer, a curable composition, and a resin member.

### Background Art

A (meth)acrylate-based polymerizable monomer may be utilized in a wide range of fields, such as a dental material like a dental curable composition or a dental adhesive, an optical material, a printing plate, a photoresist material, a paint, an adhesive, an ink, and a stereolithographic resin (for example, Patent Literatures 1 to 4). In particular, as a (meth)acrylate-based polymerizable monomer from which a cured product excellent in mechanical strength is obtained, there are known a polymerizable monomer having a bisphenol A skeleton typified by bisphenol A diglycidyl di(meth)acrylate (Bis-GMA) exemplified in Patent Literatures 2 and 3, and a polymerizable monomer having a biphenyl skeleton exemplified in Patent Literatures 4 and 5.

### Citation List

### Patent Literature

[PTL 1] JP 2008-534256 A
[PTL 2] JP 2007-126417 A
[PTL 3] JP 09-157124 A
[PTL 4] JP 05-170705 A
[PTL 5] JP 63-297344 A

### Summary of Invention

### Technical Problem

Meanwhile, from the viewpoint of facilitating the handling property of a polymerizable monomer, it is advantageous that the polymerizable monomer is a liquid or liquid-like substance having low viscosity under a room temperature environment. For example, in general, in many cases, the polymerizable monomer is used as a mixed composition having mixed therein other components depending on various use applications, rather than being used alone. In such cases, when the polymerizable monomer is a liquid or liquid-like substance having low viscosity, its blending with other components is extremely easy.

However, Bis-GMA exemplified in Patent Literatures 2 and 3 has extremely high viscosity under a room temperature environment, and the polymerizable monomer exemplified in Patent Literature 4 is even a solid under a room temperature environment, and besides, is poor in mechanical strength. In addition, the polymerizable monomer exemplified in Patent Literature 5 is also poor in mechanical strength, though having relatively low viscosity.

The present invention has been made in view of the above-mentioned circumstances, and an object of the present invention is to provide a polymerizable monomer excellent in mechanical strength of its cured product and excellent also in handling property with low viscosity even under a room temperature environment, and a method of producing the polymerisable monomer, a curable composition and a resin member each using the polymerizable monomer.

### Solution to Problem

The above-mentioned object can be achieved by the polymerizable monomer according to claim 1.

A polymerizable monomer according to one embodiment of the present invention is preferably represented by the following general formula (2): 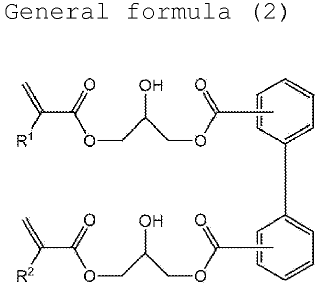 in the general formula (2), R¹ and R² are the same as those shown in the general formula (1).

A polymerizable monomer according to another embodiment of the present invention is preferably represented by the following general formula (3): 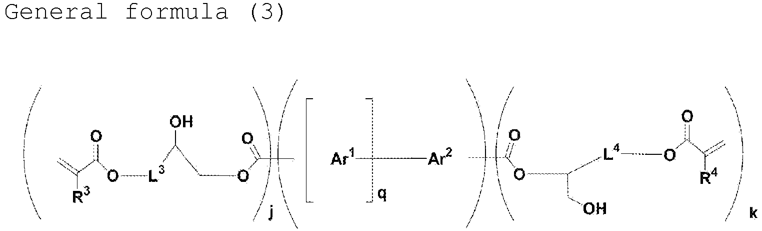 in the general formula (3), Ar¹ and Ar² are the same as those shown in the general formula (1) except that the valence may be only divalence, q is the same as that shown in the general formula (1), L³ and L⁴ each represent a divalent hydrocarbon group having a main chain with a number of atoms within a range of from 1 to 8, and may be identical to or different from each other, R³ and R⁴ each represent hydrogen or a methyl group, j represents 0, 1, or 2, k represents 0, 1, or 2, and j+k=2.

In a polymerizable monomer according to another embodiment of the present invention, a combination (j, k) of values j and k shown in the general formula (3) preferably includes a combination selected from the group consisting of (1, 1) and (0, 2) .

A polymerizable monomer according to another embodiment of the present invention preferably includes two or more kinds of structural isomers in each of which a combination (j, k) of values j and k shown in the general formula (3) is selected from the group consisting of (2, 0), (1, 1), and (0, 2).

In a polymerizable monomer according to another embodiment of the present invention, an average value of values k in all molecules of the polymerizable monomer is preferably 0.05 or more and less than 2.0.

The above-mentioned objection is also achieved by the method of producing a polymerizable monomer according to claim 10, the curable composition according to claim 13, and the resin member according to claim 14.

### Advantageous Effects of Invention

According to the present invention, the polymerizable monomer excellent in mechanical strength of its cured product and excellent also in handling property with low viscosity even under a room temperature environment, and the method of producing the polymerisable monomer, the curable composition and the resin member each using the polymerizable monomer can be provided.

### Description of Embodiments

A polymerizable monomer according to this embodiment is represented by the following general formula (1): 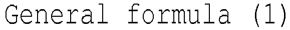 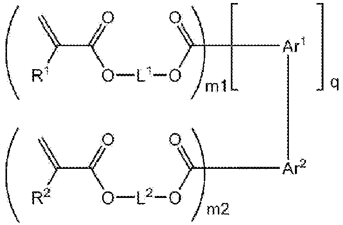 in the general formula (1), Ar¹ and Ar² each represent a substituted or unsubstituted aromatic group having a valence selected from divalence or trivalence, and may be identical to or different from each other, L¹ and L² each represent a divalent substituted hydrocarbon group having a main chain with a number of atoms within a range of from 2 to 10 and containing at least one hydroxy group, and may be identical to or different from each other, R¹ and R² each represent hydrogen or a methyl group, q is 1, and m1 and m2 each represent 1 or 2. The polymerizable monomer represented by the general formula (1) may be an isomeric mixture containing two or more kinds of isomers.

The polymerizable monomer according to this embodiment is excellent in mechanical strength of its cured product, and is also excellent in handling property because of having low viscosity even under a room temperature environment. The reasons why such effects are obtained are not clear, but the inventors of the present invention presume as follows. First, a possible reason for being excellent in mechanical strength of the cured product is that the polymerizable monomer has structure containing an aromatic group having high rigidity (Ar¹ or Ar¹-Ar²) at the central portion of the molecule. Further, as described later, two or more hydroxy groups are contained in the molecule, and hence the formation of a loose and dense hydrogen bonding network between molecules by the hydroxy groups is also considered to contribute to maintaining the mechanical strength of the cured product.

In addition, in general, a compound having a plurality of hydroxy groups in the molecule forms intermolecular hydrogen bonding, and as a result, is liable to have increased viscosity. However, a possible reason why the polymerizable monomer according to this embodiment, which has a plurality of hydroxy groups in the molecule, shows low viscosity even under a room temperature environment is that the hydrogen of the hydroxy groups constituting each of the divalent groups L¹ and L² easily forms intramolecular hydrogen bonding between itself and the oxygen of a carbonyl group constituting an ester bond directly bonded to the aromatic group Ar¹ or Ar² as shown in the structural formula exemplified below. The structural formula exemplified below is an example of the general formula (1) where Ar¹ and Ar²=phenylene group, L¹ and L²=-CH₂CH(OH)CH₂-, R¹ and R²=methyl group, q=1, and m1 and m2=1.

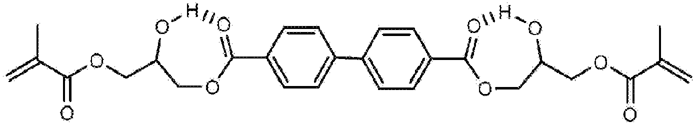

That is, when intramolecular hydrogenbonding is formed, the formation of intermolecular hydrogen bonding is suppressed as a result, to thereby decrease the viscosity. In addition, as compared to the case where no intramolecular hydrogen bonding is formed, in the case where intramolecular hydrogen bonding is formed, distortion occurs in benzoate structure formed of the benzene ring constituting the aromatic group Ar¹ or Ar² and the ester bond directly bonded thereto, and thus the symmetry of the molecular structure at the central portion of the molecule decreases. Accordingly, it is considered that the crystallinity of the molecule decreases to promote not only liquefaction but also the decrease in viscosity. The formation of intramolecular hydrogen bonding weakens intermolecular bonding, and hence has a risk of causing a decrease in mechanical strength of the cured product as well. However, in the polymerizable monomer according to this embodiment, the hydroxy groups are, more accurately, considered to contribute to intramolecular hydrogen bonding to a relatively higher degree than to intermolecular hydrogen bonding, and are considered to contribute also to forming a loose hydrogen bonding network between molecules. In addition, the polymerizable monomer according to this embodiment contains a plurality of hydroxy groups in the molecule, and hence facilitates the formation of a hydrogen bonding network having high density between molecules. That is, it is considered that the mechanical strength of the cured product is easily maintained in the case of having a plurality of hydroxy groups in the molecule as in the polymerizable monomer according to this embodiment as compared to the case where one hydroxy group is contained in the molecule.

The above-mentioned findings have been found by the inventors of the present invention on the basis of comparison with the fact that Bis-GMA exemplified in Patent Literatures 2 and 3 is a highly viscous liquid under room temperature, the fact that the polymerizable monomer exemplified in Patent Literature 4 shows a solid state under room temperature, and the molecular structures of these polymerizable monomers. That is, the inventors of the present invention have considered that: the reason why Bis-GMA having bisphenol A structure having high rigidity at the central portion of the molecule shows high viscosity is that the hydrogen of the hydroxy groups contained in the molecule of Bis-GMA forms hydrogen bonding intermolecularly rather than intramolecularly; and the reason why the polymerizable monomer exemplified in Patent Literature 4 shows a solid state is that the dibenzoate structure having high rigidity constituting the central portion of the molecule (structure in which ester bonds are bonded to both ends of biphenyl) has high symmetry and is liable to crystallize. In addition, from those facts, it is considered that in order to obtain a polymerizable monomer having low viscosity while ensuring excellent mechanical strength of its cured product, it is important to (1) suppress intermolecular hydrogen bonding and (2) decrease the symmetry of molecular structure mainly formed of an aromatic group occupying the central portion of the molecule to decrease crystallinity.

In view of the foregoing, the inventors of the present invention have found, as a polymerizable monomer having molecular structure capable of complexly and synergistically achieving the above-mentioned (1) and (2), the polymerizable monomer represented by the general formula (1), which has benzoate structure at the central portion of the molecule and which also has hydroxy groups that decrease the symmetry of the benzoate structure by intramolecular hydrogen bonding while hardly contributing to intermolecular hydrogen bonding.

Next, the polymerizable monomer represented by the general formula (1) is described in more detail. First, in the general formula (1), Ar¹ and Ar² each represent an aromatic group having a valence selected from divalence or trivalence, and specific examples thereof include divalent or trivalent benzenes represented by the following structural formulae Ar-a1 to Ar-a2, divalent or trivalent naphthalenes represented by the following structural formulae Ar-a4 to Ar-a5, and divalent to trivalent anthracenes represented by the following structural formulae Ar-a7 to Ar-a8. In these structural formulae, bonding sites may each be located on any carbon of the benzene rings constituting the aromatic groups Ar¹ and Ar² (except for carbon forming a condensed portion between benzene rings) . For example, in the case of the structural formula Ar-a1 (divalent benzene), the two bonding sites may be located at ortho positions, meta positions, or para positions.

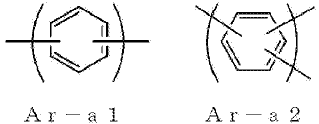

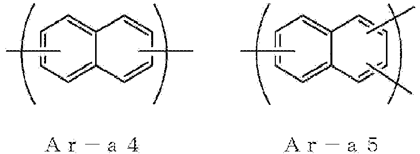

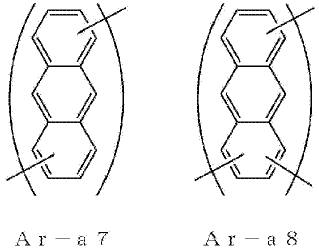

Here, q=1, and Ar¹ and Ar² are bonded to each other by a σ-bond to constitute structure "Ar¹-Ar²", and the remaining bonding sites of Ar¹ and Ar² not involved in the formation of the σ-bond are each bonded to the carbon of an ester bond.

In the case of (q, m1, m2)=(1, 1, 1), Ar¹ and Ar² each have two bonding sites. In the case of (q, m1, m2)=(1, 2, 1), Ar¹ has three bonding sites, and Ar² has two bonding sites. In the case of (q, m1, m2)=(1, 1, 2), Ar¹ has two bonding sites, and Ar² has three bonding sites. In the case of (q, m1, m2)=(1, 2, 2), Ar¹ and Ar² each have three bonding sites.

Ar¹ and Ar² may be identical to or different from each other. (q, m1, m2) is preferably (1, 1, 1), more preferably (1, 1, 1).

In addition, the aromatic groups Ar¹ and Ar² may each have a substituent, and in this case, the hydrogen of the benzene rings constituting the aromatic groups Ar¹ and Ar² may be substituted by any other substituent. The substituent of each of the aromatic groups Ar¹ and Ar² is not particularly limited as long as the substituent does not contain, at an end thereof, any of the reactive groups shown on the left side of the general formula (1) (that is, an acrylic group or a methacrylic group) . A substituent having a total number of atoms constituting the substituent (number of atoms) within the range of from 1 to 60 may be appropriately selected. Specific examples thereof may include a monovalent hydrocarbon group having 1 to 20 carbon atoms, -COOR³, -OR³, a halogen group, an amino group, a nitro group, and a carboxyl group. R³ is the same as the monovalent hydrocarbon group having 1 to 20 carbon atoms . In addition, examples of the monovalent hydrocarbon group having 1 to 20 carbon atoms may include: linear or branched hydrocarbon groups, such as a methyl group and an ethyl group; alicyclic hydrocarbon groups, such as a cyclohexyl group; a phenyl group; and heterocyclic groups, such as monovalent furan.

L¹ and L² each represent a divalent substituted hydrocarbon group having a main chain with a number of atoms within the range of from 2 to 10 and containing at least one hydroxy group, and may be identical to or different from each other. The number of hydroxy groups contained in the hydrocarbon group only needs to be at least 1, and may be 2 or more, but is generally preferably 1. That is, in the case of m1 and m2=1, two hydroxy groups are preferably contained in the molecule. The number of atoms of the main chain falls within more preferably the range of from 2 to 6, still more preferably the range of from 2 to 3. Particularly when the number of atoms of the main chain is set to fall within the range of from 2 to 3, the bending strength of the cured product is further increased with ease.

The atoms constituting the main chain are basically constituted of carbon atoms and all the atoms may be carbon atoms, but part of the carbon atoms constituting the main chain may be substituted by a heteroatom. Examples of the heteroatom may include an oxygen atom, a nitrogen atom, a sulfur atom, and a silicon atom. When the main chain contains an oxygen atom as the heteroatom, the main chain may have an ether bond or an ester bond introduced thereinto . The number of heteroatoms that may be introduced into the main chain is preferably 1 or 2. When the number of atoms of the main chain is 2, the number of heteroatoms that may be introduced into the main chain is 1.

In addition, of the atoms constituting the main chain, at least any one atom (generally a carbon atom) has bonded thereto a hydroxy group or a monovalent hydrocarbon group having a hydroxy group. The monovalent hydrocarbon group having a hydroxy group has a number of carbon atoms within preferably the range of from 1 to 3, more preferably the range of from 1 to 2. Specific examples of the monovalent hydrocarbon group having a hydroxy group include -CH₂OH, -CH₂CH₂OH, and -CH(CH₃)OH. Of those, -CH₂OH or -CH(CH₃)OH is more preferred.

In addition, of the atoms constituting the main chain, at least any one atom (generally a carbon atom) may have bonded thereto a substituent other than a hydroxy group and a monovalent hydrocarbon group having a hydroxy group. Examples of such substituent may include an alkyl group having 1 to 3 carbon atoms, such as a methyl group, a halogen, -COOR⁴, and -OR⁴. R⁴ is the same as the alkyl group having 1 to 3 carbon atoms.

Specific examples of L¹ and L² may include the following structural formulae L-a1 to L-a37. Of the two bonding sites shown in each of these structural formulae, the bonding site marked with symbol "*" means a bonding site to be bonded to the oxygen atom of the ester bond constituting the benzoate structure at the central portion of the molecule.

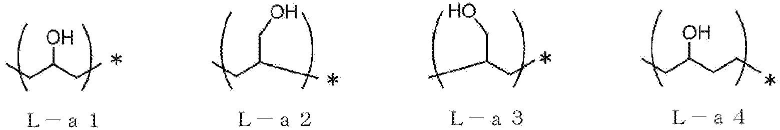

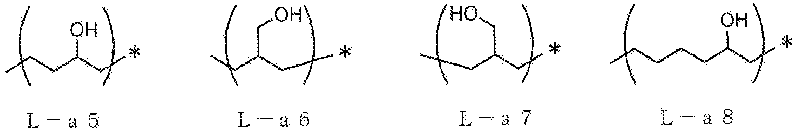

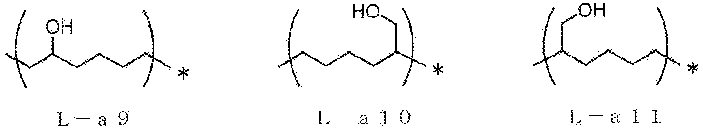

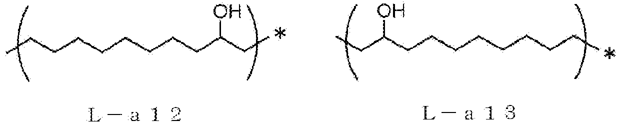

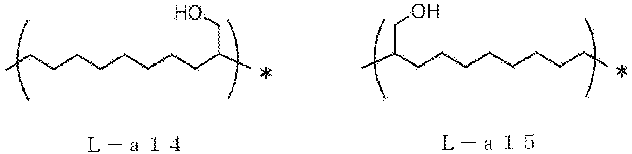

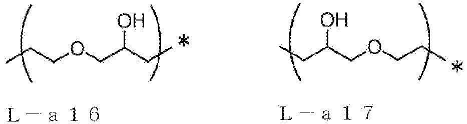

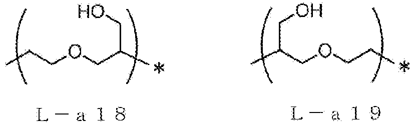

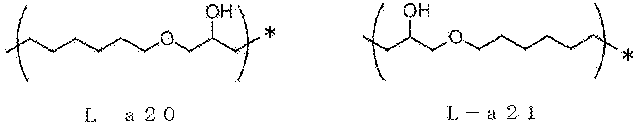

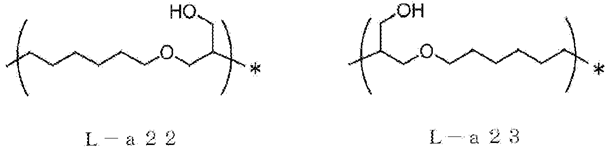

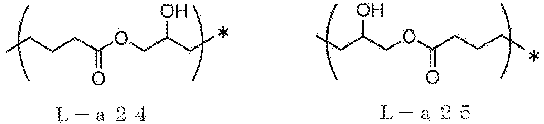

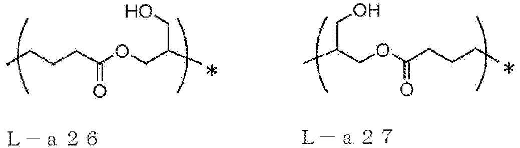

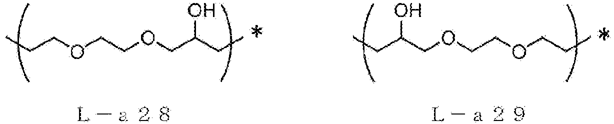

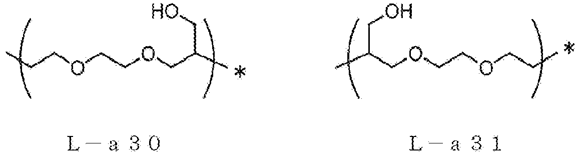

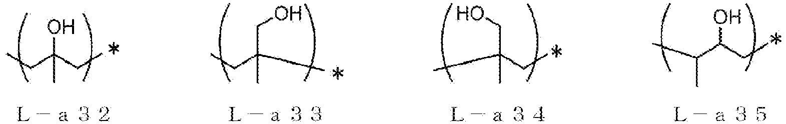

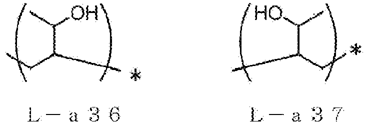

The polymerizable monomer according to this embodiment, which is represented by the general formula (1), is particularly preferably a polymerizable monomer represented by the following general formula (2) . The general formula (2) represents the structure of the general formula (1) in the case where q=1, m1 and m2=1, Ar¹ and Ar²=-C₆H₄- (structural formula Ar-a1), and L¹ and L²=-CH₂CH(OH)CH₂- (structural formula L-a1).

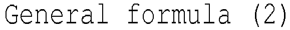

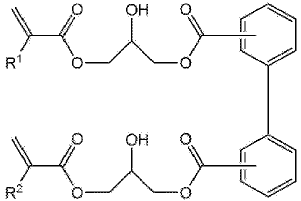

In addition, the polymerizable monomer according to this embodiment is preferably a polymerizable monomer represented by the following general formula (3). In the general formula (3), Ar¹ and Ar² are the same as those shown in the general formula (1) except that the valence may be only divalence, q is the same as that shown in the general formula (1), L³ and L⁴ each represent a divalent hydrocarbon group having a main chain with a number of atoms within the range of from 1 to 8, and may be identical to or different from each other, and R³ and R⁴ each represent hydrogen or a methyl group. In addition, j represents 0, 1, or 2, k represents 0, 1, or 2, and j+k=2. The general formula (3) represents the structure (bifunctional structure) of the general formula (1) in the case where m1 and m2=1, L¹=-L³-CH(OH)CH₂- or -CH(CH₂OH)-L⁴-, L²=-L³-CH(OH)CH₂- or -CH(CH₂OH) -L⁴-, R¹ corresponds to R³ or R⁴, and R² corresponds to R³ or R⁴. In addition, in the general formula (3), the groups shown in parentheses on both left and right sides may each be bonded to any of the two bonding sites of the group shown in the center: -[Ar¹]_{q}-Ar²-. That is, depending on the values of j and k, the group shown in the parentheses on the left side in the general formula (3) is bonded to both sides of the group shown in the center in some cases, and the group shown in the parentheses on the right side in the general formula (3) is bonded to both sides of the group shown in the center in other cases.

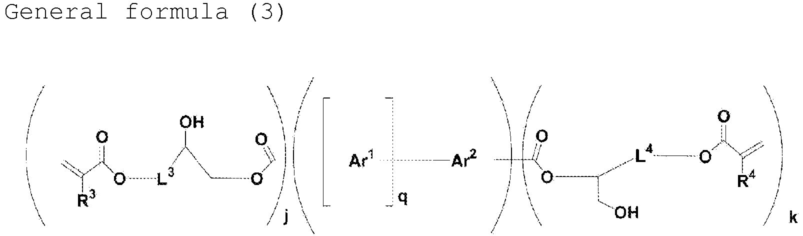

In each of L³ and L⁴, the atoms constituting the main chain are basically constituted of carbon atoms and all the atoms may be carbon atoms, but part of the carbon atoms constituting the main chain may be substituted by a heteroatom. Examples of the heteroatom may include an oxygen atom, a nitrogen atom, a sulfur atom, and a silicon atom. When the main chain contains an oxygen atom as the heteroatom, the main chain may have an ether bond or an ester bond introduced thereinto. The number of heteroatoms that may be introduced into the main chain is preferably 1 or 2. When the number of atoms of the main chain is 2, the number of heteroatoms that may be introduced into the main chain is 1.

In addition, in each of L³ and L⁴, the number of atoms of the main chain only needs to be from 1 to 8, but is preferably from 1 to 5, more preferably from 1 to 3, most preferably 1. Specific examples of L³ and L⁴ include: an alkylene group having a main chain with 1 to 8 carbon atoms, such as a methylene group, an ethylene group, a n-propylene group, or a n-butylene group; and a group obtained by partially or completely substituting the main chain of the alkylene group with an ether bond or an ester bond (provided that the number of atoms of the main chain of the alkylene group is 2 or more).

As the combination (j, k) of the values j and k shown in the general formula (3), there are given (2, 0), (1, 1), and (0, 2). Of those, from the viewpoint that the suppression of the decomposition of the polymerizable monomer molecule can be expected, (1, 1) and (0, 2) are more preferred. With regard to the reason for this, the inventors of the present invention assume as follows.

Here, the formula shown below represents an example of a mechanism for a reaction of the molecule of the polymerizable monomer represented by the general formula (3) where (j, k)=(1, 1), q=1, Ar¹ and Ar²=-C₆H₄-, L³ and L⁴=-CH₂-, and R³ and R⁴=-CH₃ in the presence of a water molecule. The polymerizable monomer according to this embodiment has molecular structure having an ester bond directly bonded to an aromatic group at the central portion of the molecule. One possible reaction mechanism by which the polymerizable monomer having such molecular structure is decomposed is, for example, a nucleophilic reaction of a water molecule on an ester bond directly bonded to an aromatic group (hydrolysis reaction) (Route B below) . However, when a primary alcohol contained in the same molecule is present in the immediate vicinity of the ester bond directly bonded to the aromatic group, the primary alcohol and the ester bond cause a cyclization reaction in the molecule, to thereby temporarily form cyclic structure (Route A below). The cyclization reaction is reversible, and hence the cyclic structure easily returns to the original linear structure immediately, but the cyclization reaction inhibits the above-mentioned nucleophilic reaction (hydrolysis reaction) or the like. Accordingly, it is assumed that the decomposition of the polymerizable monomer molecule is inhibited. In this case, decomposition deterioration of the polymerizable monomer or a composition containing the polymerizable monomer is suppressed (in other words, storage stability is improved), and hence the initial performance of any of various compositions using the polymerizable monomer immediately after production can be stably maintained over a long period of time. For example, in the case of a dental composition (e.g., a dental filling restorative material or a dental adhesive) using the polymerizable monomer, its adhesive strength immediately after production and the mechanical strength of its cured product are easily maintained even after long-term storage.

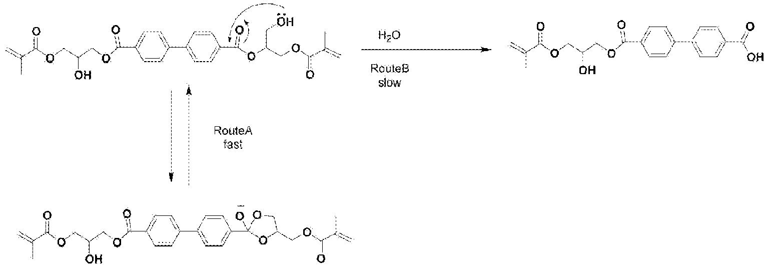

In addition, the polymerizable monomer according to this embodiment preferably contains two or more kinds of structural isomers in each of which the combination (j, k) of the values j and k shown in the general formula (3) is selected from the group consisting of (2, 0), (1, 1), and (0, 2). When the polymerizable monomer contains two or more kinds of structural isomers for the combination of (j, k) shown in the general formula (3), it is easy to improve the mechanical strength of the cured product and the storage stability in a well-balanced manner. In this case, the average value of the values k in all molecules of the polymerizable monomer preferably falls within the range of from 0.05 or more to less than 2.0 (in other words, the average value of the values j falls within the range of from more than 0 to 1.95 or less) . Further, the lower limit of the average value of the values k is more preferably 0.1 or more, and the upper limit of the average value of the values k is more preferably 1.7 or less, still more preferably 1.5 or less. In order to improve the mechanical strength of the cured product and the storage stability in a well-balanced manner, the value k=2 (state of not containing structural isomers) is suitable as well as the case where the average value of the values k is set to the range of from 0.05 or more to less than 2.0. In the state of containing two or more kinds of structural isomers, the mechanical strength in the initial state without undergoing a certain storage period can be made higher than in the case of the value k=2 (state of not containing structural isomers). In this regard, it is more advantageous that the average value of the values k is smaller within the range in which the average value of the values k is not less than 0.05.

When only the above-mentioned reaction mechanism is taken into consideration, it is predicted that as the average value of the values k increases, the storage stability also improves. However, an investigation made by the inventors of the present invention has found that a remarkable improving effect on the storage stability is obtained even when the average value of the values k is about 0.1 (when the presence ratio of the primary alcohol present in the vicinity of an ester bond directly bonded to an aromatic group is small in the whole polymerizable monomer) (see Examples to be described later) . From those results, it is assumed that an unexpected factor other than the above-mentioned reaction mechanism exists for the improvement of the storage stability.

The polymerizable monomer according to this embodiment may be synthesized by appropriately combining known starting raw materials and a known synthesis reaction method, and its production method is not particularly limited. For example, when the polymerizable monomer represented by the general formula (3) is produced, there may be utilized a production method including at least a reaction step of allowing a compound represented by the following general formula (4) and a compound represented by the following general formula (5) to react with each other. In this case, a polymerizable monomer containing two or more kinds of structural isomers selected from the group consisting of compounds represented by the following general formulae (6) to (8) may be produced.

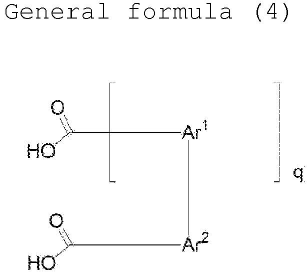

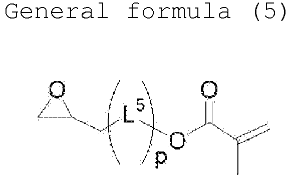

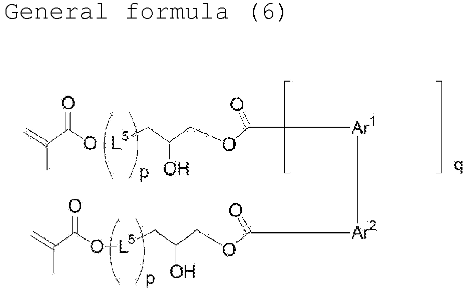

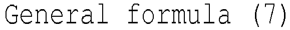

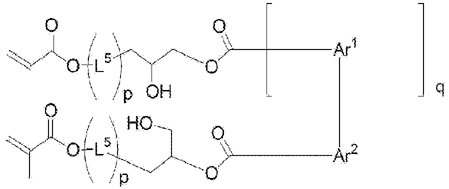

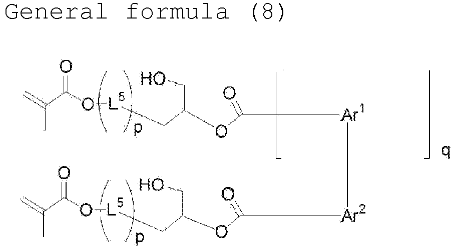

In the general formulae (4) to (8), Ar¹, Ar², and q are the same as those shown in the general formula (3), and L⁵ represents a divalent substituted or unsubstituted hydrocarbon group having a main chain with a number of atoms of from 1 to 7. In addition, p represents 0 or 1. Here, the average value of the values k, in other words, the presence ratio among the structural isomers represented by the general formulae (6) to (8) can be easily adjusted by appropriately selecting synthesis conditions. In addition, as necessary, through purification treatment after the synthesis, the average value of the values k (presence ratio among the structural isomers represented by the general formulae (6) to (8)) maybe adjusted so as to be closer to a desired value.

In L⁵ shown in the general formulae (5) to (8), the atoms constituting the main chain are basically constituted of carbon atoms and all the atoms may be carbon atoms, but part of the carbon atoms constituting the main chain may be substituted by a heteroatom. Examples of the heteroatom may include an oxygen atom, a nitrogen atom, a sulfur atom, and a silicon atom. When the main chain contains an oxygen atom as the heteroatom, the main chain may have an ether bond or an ester bond introduced thereinto . The number of heteroatoms that may be introduced into the main chain is preferably 1 or 2. When the number of atoms of the main chain is 2, the number of heteroatoms that may be introduced into the main chain is 1.

In addition, in L⁵, the number of atoms of the main chain only needs to be from 1 to 7, but is preferably from 1 to 4, more preferably 1 or 2. Specific examples of L⁵ include: an alkylene group having a main chain with 1 to 7 carbon atoms, such as a methylene group, an ethylene group, a n-propylene group, and a n-butylene group; and a group obtained by partially or completely substituting the main chain of the alkylene group with an ether bond or an ester bond (provided that the number of atoms of the main chain of the alkylene group is 2 or more).

In the general formula (6), L⁵'s may be identical to or different from each other. The same applies to the general formulae (7) and (8). When L⁵'s are set to be different from each other, two or more kinds of compounds different from each other in L⁵ may be used as the compound represented by the general formula (5) to be used for the synthesis. In addition, p preferably represents 0.

If necessary, after the polymerizable monomer containing two or more kinds of structural isomers has been obtained by the above-mentioned production method, only a polymerizable monomer substantially not containing structural isomers (e.g., the polymerizable monomer represented by the general formula (6)) may be isolated and purified. However, the polymerizable monomer to be obtained by the isolation and purification tends to be poor in terms of compatibility between the mechanical strength of the cured product and the storage stability as compared to the polymerizable monomer containing two or more kinds of structural isomers before the isolation and purification treatment. In addition, the isolation and purification treatment is further needed in the production of the polymerizable monomer, and hence is liable to be disadvantageous in terms of cost. Accordingly, from those viewpoints, the isolation and purification treatment is preferably avoided.

-Composition, Curable Composition, and Resin Member-The polymerizable monomer according to this embodiment may be utilized as a composition containing the polymerizable monomer. However, from the viewpoint of utilization in various applications, the polymerizable monomer according to this embodiment is also suitably used as a composition containing the polymerizable monomer according to this embodiment and any other material. In this case, a polymerization initiator may be further added to a composition containing at least the polymerizable monomer according to this embodiment, to thereby formulate a curable composition. When the composition containing the polymerizable monomer according to this embodiment is a composition containing no polymerization initiator, this composition (agent A) may be used in combination with another composition (agent B) containing a polymerization initiator. In this case, a cured product may be obtained by polymerizing a mixture of the agent A and the agent B (first curing mode).

In addition, in the case of the curable composition (agent C) containing the polymerizable monomer according to this embodiment and a polymerization initiator, a cured product may be obtained by curing the agent C alone (second curing mode) . However, as necessary, the agent C may be used in combination with another composition (agent D). In this case, a cured product may be obtained by curing a mixture of the agent C and the agent D (third curing mode). In addition, a cured product may also be obtained by applying the agent D onto a solid surface, and further placing the agent C thereon, followed by curing. In this case, when the agent D functions as an adhesive and/or a pretreatment material for improving conformability between the agent C and the solid surface, a cured product in a state of being bonded to the solid surface can be obtained (fourth curing mode). In addition, when the agent D functions as a release agent, after curing, the cured product can be collected by being easily separated from the solid surface without any damage to the surface of the cured product (fifth curing mode).

As a composition that may be suitably utilized in each of the first and third curing modes, for example, there is given a two-component system adhesive. As a composition that may be suitably utilized in the second curing mode, for example, there is given a resin raw material to be used for a 3D printer utilizing a layered manufacturing method. As a composition that maybe suitably utilized in the fourth curing mode, for example, there is given a dental material (in particular, a composite resin to be used for filling the cavity of a tooth) . As a composition that may be suitably utilized in the fifth curing mode, for example, there is given a resin raw material to be used for the production of a molded product using a mold.

In addition, the other material to be used together with the polymerizable monomer according to this embodiment is not particularly limited, and may be appropriately selected depending on applications of the composition or curable composition containing the polymerizable monomer according to this embodiment. Specific examples of the other material may include a polymerizable monomer other than the polymerizable monomer according to this embodiment, a low-molecular organic compound having no reactivity, a resin, afiller, an organic-inorganic composite material, various additives other than the above-mentioned polymerization initiator, and a solvent. Two or more kinds of those materials may be used in combination.

However, the other material to be used in combination with the polymerizable monomer according to this embodiment is particularly preferably another polymerizable monomer. As the other polymerizable monomer, a known polymerizable monomer may be used without any limitation. However, in consideration of the fact that the polymerizable monomer according to this embodiment has a feature of having low viscosity under a room temperature environment, it is preferred that the other polymerizable monomer also have such relatively low viscosity as not to offset the feature. From such viewpoint, the viscosity of the other polymerizable monomer is preferably 150 mPa·S or less at room temperature (25°C). Further, in consideration of the ease of ensuring compatibility when reactive groups located at both ends of a molecule are identical or similar to each other, the other polymerizable monomer is preferably a bifunctional (meth)acrylate-based polymerizable monomer.

Examples of the bifunctional (meth)acrylate-based polymerizable monomer having the above-mentioned viscosity characteristic may include polyalkylene glycol dimethacrylates, such as triethylene glycol dimethacrylate (3G) (more specifically, polyethylene glycol dimethacrylate having a polymerization degree of alkylene glycol units of 1 or more and 14 or less, polypropylene glycol dimethacrylate having a polymerization degree of alkylene glycol units of 1 or more and 7 or less, polymethylene glycol dimethacrylate having 2 to 10 carbon atoms, and the like), neopentyl glycol dimethacrylate, tricyclodecane dimethanol dimethacrylate (TCD), and 1,9-nonanediol dimethacrylate (ND). In addition, from the viewpoint of compatibility, the other polymerizable monomer is preferably one having a polyalkylene glycol chain, more preferably one having a polyethylene glycol chain. This is because the polyalkylene glycol chain has high affinity for the benzoate structure constituting the central portion of the molecule of the polymerizable monomer according to this embodiment (that is, a non-hydrogen-bonding polar group). In consideration of the respects described above, the other polymerizable monomer is preferably polyalkylene glycol dimethacrylate, particularly preferably triethylene glycol dimethacrylate.

In the description of the present application, the viscosity of the polymerizable monomer means a value measured at 25°C using an E-type viscometer.

In addition, the other polymerizable monomer may be selected depending on applications of the composition or curable composition using the polymerizable monomer according to this embodiment, or a resin member including the cured product obtained by using the polymerizable monomer according to this embodiment. For example, when the composition or curable composition using the polymerizable monomer according to this embodiment is used as a dental material, a polymerizable monomer, such as triethylene glycol dimethacrylate, tricyclodecanedimethanol dimethacrylate, or 1,9-nonanediol dimethacrylate, is preferably used. When the polymerizable monomer according to this embodiment and the other polymerizable monomer are used as a mixture, the polymerizable monomer according to this embodiment accounts for preferably 20 mass% or more, more preferably 30 mass% or more, particularly preferably 60 mass% or more of all polymerizable monomers.

As the polymerization initiator to be used in the curable composition using the polymerizable monomer according to this embodiment, a known polymerization initiator may be used, and any of various polymerization initiators, such as a radical-type, cation-type, or anion-type photopolymerization initiator, and an azo-based or peroxide-based thermal polymerization initiator, may be appropriately utilized. For example, when polymerization and curing are performed by light irradiation, a photopolymerization initiator, such as camphorquinone or ethyl p-N,N-dimethylaminobenzoate, may be used. In addition, two or more kinds of those polymerization initiators may be used in combination. Further, any other additive, such as a polymerization inhibitor or a sensitizer, may be used in combination with the polymerization initiator.

In addition, it is also suitable to add a filler to the composition or curable composition using the polymerizable monomer according to this embodiment. The use of the filler can further increase the suppressive effect on polymerization shrinkage. In addition, through the use of the filler, the operability of the composition or curable composition before curing can be improved, or mechanical properties after curing can be improved. As the filler, for example, an inorganic filler formed of particles of an inorganic oxide, such as amorphous silica, silica-titania, silica-zirconia, silica-titania-barium oxide, quartz, or alumina, may be used, and an organic filler or an organic-inorganic composite filler may also be used. In addition, the particle diameter and shape of the filler are not particularly limited, but for example, particles each having a spherical shape or an irregular shape and having an average particle diameter of from about 0.01 µm to about 100 µm may be appropriately used depending on purposes. In addition, any such filler may be treated with a surface treatment agent typified by a silane coupling agent from the viewpoint of improving conformability with the polymerizable monomer according to this embodiment and the other material, such as the other polymerizable monomer, to be used in combination as necessary, to thereby improve mechanical strength and water resistance.

The polymerizable monomer according to this embodiment may be used in various applications, such as a dental material like a dental curable composition or a dental adhesive, an optical material, a printing plate, a photoresist material, a paint, an adhesive, an ink, a stereolithographic resin, a woodworking coating, a hard coating, a film coating, a paper coating, an optical fiber coating, a PVC floor coating, a ceramic wall coating, a resin hard coat, a metallized base coat, a release coating, a metal coating, a glass coating, an inorganic material coating, an elastic coating, a planographic ink, a metal can ink, a screen printing ink, a gravure varnish, calendering, a paint, a sealant, an adhesive for paper, an adhesive for a film, an adhesive for woodworking, an adhesive for an inorganic material, an adhesive for a plastic, a solvent-based adhesive, a water-based adhesive, a hot-melt adhesive, a reaction-type adhesive, a pressure-sensitive adhesive, a sealing agent, a gloss coating agent, an OP varnish, an etching resist, a solder-resist, a dry film, an interlayer adhesion agent for a build-up circuit board, a photosensitive material, a semiconductor photoresist, a printed wiring board resist, a photosensitive flexographic plate, a photosensitive resin relief printing plate, a PS plate, a CTP plate, PDP, a color resist for a color filter, a black matrix material, a color filter overcoat agent, a photoresist for a liquid crystal, a seal agent, an injection port-sealing agent, a prism sheet, potting, a moisture-proof coat, a protective coat, an ink binder, an adhesive binder, a sealing material, a primer for a base material, e.g., a plastic, a metal, or paper, an agent for temporary fixing, a stereolithographic material, a cast resin, a reactive diluent, temporary anticorrosion of iron, a metal primer/overcoat, a primer for a metal coil/sheet, an adhesive tape, a transfer film, a plastic lens, and a glass lens. In addition, depending on its use applications, the polymerizable monomer according to this embodiment may also be used by being mixed with various components as necessary.

Meanwhile, the polymerizable monomer according to this embodiment andBis-GMAare similar to each other in that the structures of reactive groups at their molecular ends are identical (methacrylic group and methacrylic group) or substantially identical (acrylic group and methacrylic group), and that the side chain portion linking the central portion of the molecule mainly formed of an aromatic group and the reactive group has a hydroxy group. However, when attention is focused on the molecular structure of the central portion of the molecule, the polymerizable monomer according to this embodiment has benzoate structure, which has higher polarity than that of the bisphenol A structure constituting the central skeleton of Bis-GMA. Accordingly, when the molecule is viewed as a whole, it is considered that the polymerizable monomer according to this embodiment has higher hydrophilicity than that of Bis-GMA.

In addition, the polymerizable monomer according to this embodiment and the polymerizable monomer disclosed in Patent Literature 4 are similar to each other in having a methacrylic group or an acrylic group as a reactive group at a molecular end, and having dibenzoate structure at the central portion of the molecule. However, when attention is focused on the side chain portion linking the central portion of the molecule and the reactive group, the polymerizable monomer according to this embodiment has hydroxy groups having extremely high hydrophilicity, whereas the polymerizable monomer disclosed in Patent Literature 4 has no hydroxy group. That is, the polymerizable monomer according to this embodiment is hydrophilic, whereas the polymerizable monomer disclosed in Patent Literature 4 is hydrophobic. Further, the polymerizable monomer according to this embodiment and the polymerizable monomer disclosed in Patent Literature 5 are similar to each other in molecular structure, but are different from each other in the number of hydroxy groups contained in the molecule. That is, as compared to the polymerizable monomer disclosed in Patent Literature 5, which has one hydroxy group contained in the molecule, the polymerizable monomer according to this embodiment, which has two or more hydroxy groups contained in the molecule, is more hydrophilic.

In consideration of those respects, when compared to Bis-GMA or the polymerizable monomer disclosed in Patent Literature 4 or Patent Literature 5, the polymerizable monomer according to this embodiment may be said to be suitable and advantageous for utilization in applications requiring more hydrophilicity. For example, when the polymerizable monomer according to this embodiment is used as an adhesive, it is preferred that a surface onto which the adhesive is to be applied be hydrophilic, and when the polymerizable monomer according to this embodiment is used by being mixed with any other material, it is preferred that the other material be a hydrophilic material. When an adhesive containing the polymerizable monomer according to this embodiment is used for a hydrophilic surface, more excellent adhesive strength can be obtained as compared to the case of using an adhesive containing Bis-GMA or the polymerizable monomer disclosed in Patent Literature 4 or Patent Literature 5. In addition, when a mixed composition obtained by mixing the polymerizable monomer and a hydrophilic solid material, such as an inorganic filler, which relatively shows hydrophilicity even after subjected to surface treatment with a silane coupling agent or the like, is produced, the polymerizable monomer according to this embodiment facilitates the mixing, and moreover, allows a larger amount of a hydrophilic solid material to be blended and easily suppresses an increase in viscosity of the mixed composition along with the increase in blending amount, as compared to Bis-GMA or the polymerizable monomer disclosed in Patent Literature 4.

Meanwhile, the polymerizable monomer disclosed in Patent Literature 5, which is similar to the polymerizable monomer according to this embodiment in terms of the position and function of a hydroxy group contained in the molecule, and in terms of molecular structure, has low viscosity as compared to even Bis-GMA, and is excellent in handling property like the polymerizable monomer according to this embodiment. However, the polymerizable monomer disclosed in Patent Literature 5, which has one hydroxy group contained in the molecule, has low density of a hydrogen bonding network that can be intermolecularly formed, and hence is liable to be poor in terms of the mechanical strength of its cured product as compared to the polymerizable monomer according to this embodiment, which has two or more hydroxy groups contained in the molecule.

A non-aromatic (meth)acrylate-based polymerizable monomer having a hydroxy group, such as pentaerythritol dimethacrylate, is also useful because the polymerizable monomer is excellent in handling property with low viscosity, and is also excellent in adhesive property for a hydrophilic member surface, as compared to Bis-GMA or the polymerizable monomer disclosed in Patent Literature 4 or Patent Literature 5. However, the non-aromatic (meth)acrylate-based polymerizable monomer having a hydroxy group does not have an aromatic skeleton in the molecule, and hence is poor in mechanical strength of its cured product. In addition, such polymerizable monomer has a hydroxy group in the molecule, and hence has high hydrophilicity, but is poor in water resistance, and hence is not suitable for long-term use under water or a high-humidity environment, e.g., in an oral cavity. However, the polymerizable monomer according to this embodiment can exhibit excellent characteristics also in terms of the mechanical strength of the cured product and the water resistance as compared to the non-aromatic (meth)acrylate-based polymerizable monomer having a hydroxy group.

In consideration of the characteristics of the polymerizable monomer according to this embodiment described above, the polymerizable monomer can be widely used as a component of a dental curable composition. The polymerizable monomer according to this embodiment is suitably used as a polymerizable monomer constituting a dental material, in particular, a dental adhesive to be used for bonding onto a hydrophilic tooth surface, or a dental filling restorative material (composite resin) in which an inorganic filler is blended to further increase mechanical strength and which is required to have high affinity for the tooth surface serving as a hydrophilic adherend surface. Further, the polymerizable monomer according to this embodiment may be used also as a dental adhesive composite resin having the functions of both the dental adhesive and the composite resin. In addition, a cured product obtained by using a composition containing the polymerizable monomer according to this embodiment is excellent in mechanical strength. Accordingly, a resin member including the cured product is also suitably utilized in applications requiring mechanical strength.

### Examples

Now, in order to specifically describe the present invention, Examples and Comparative Examples are given and described, but the present invention is by no means limited thereto. Abbreviations or symbols of substances used in the preparation of samples of Examples and Comparative Examples, and their structural formulae or substance names, preparation methods for various samples, and various evaluation methods are described below.

### (1) Abbreviations or Symbols, and their Structural Formulae or Substance Names

### [First Polymerizable Monomer (Polymerizable Monomer represented by General Formula (1))]

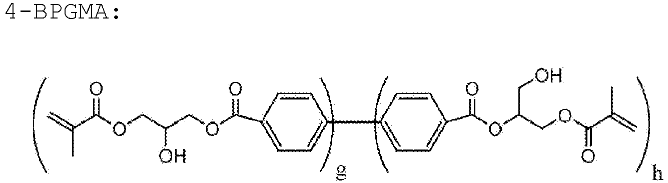

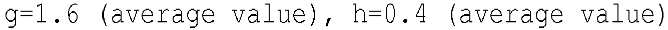

4-BPGMA is obtained as a mixture of the following compounds (a), (b), and (c), and their ratio is 65:30:5 in terms of molar ratio. In addition, the values g and h shown with the structural formula above are average values of the mixture of the compounds (a), (b), and (c). The values g and h shown with the structural formula above and the following structural formulae mean average values, but in individual molecules, the values of g and h may each take an integer value of 0, 1, or 2. In addition, except for the case where the average values of the values g and h are each 0 or 2, the structural formulae in which the average values of the values g and h are shown each mean a mixture of two kinds or three kinds of structural isomers having different combinations of integer values (g, h). Further, in the general formula (3), in the case of q=1 and Ar¹=Ar²=phenylene group, the value g is a value corresponding to the value j shown in the general formula (3), and the value h is a value corresponding to the value k shown in the general formula (3).

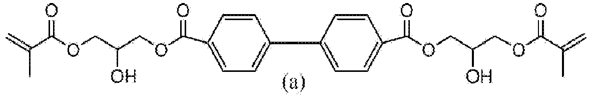

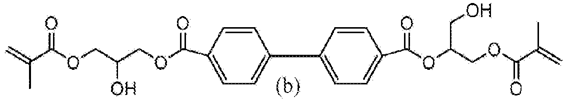

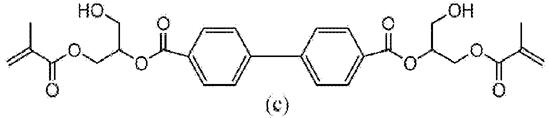

### 4-BPGMAI :

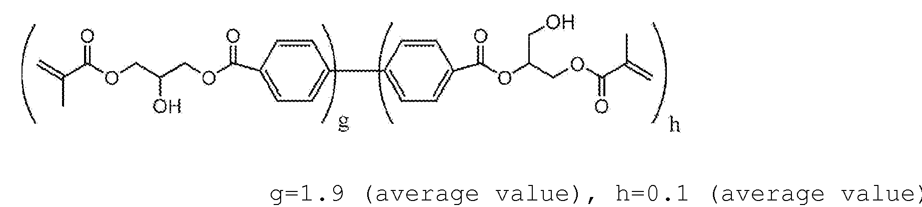

### 4-BPGMAII :

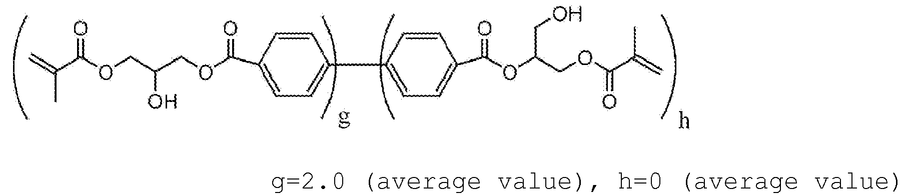

### 4-BPGMAIII :

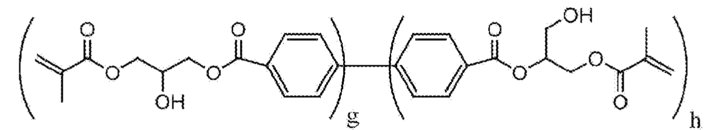

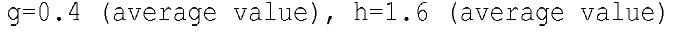

### 2-BPGMA :

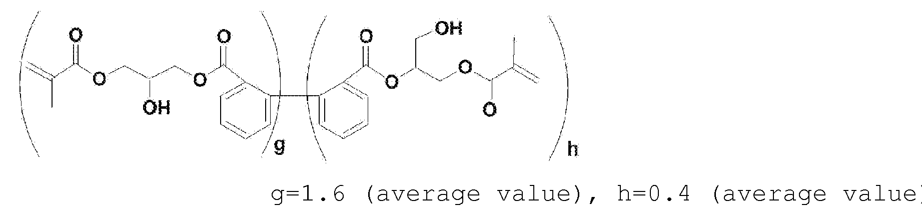

### 2-BPGMAI :

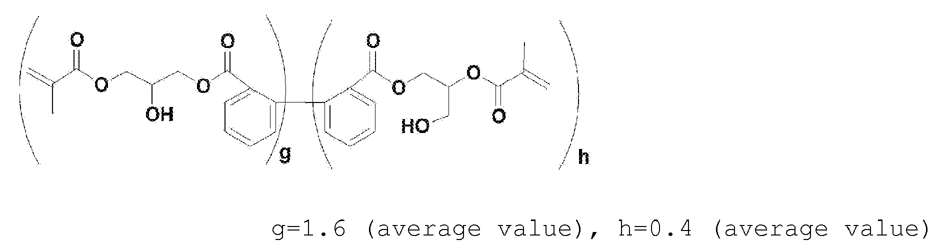

### 2-BPGMAII :

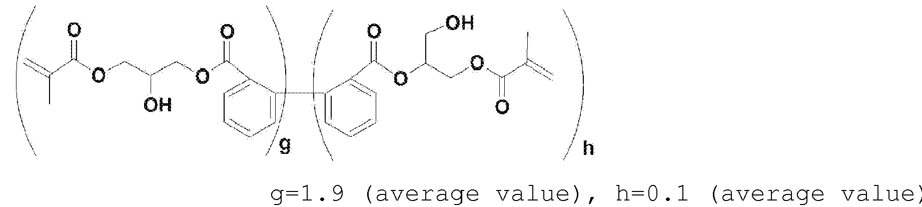

### 2-BPGMAIII :

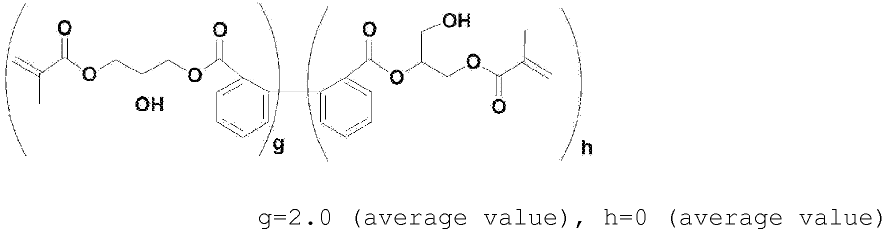

### 2-BPGMAIV :

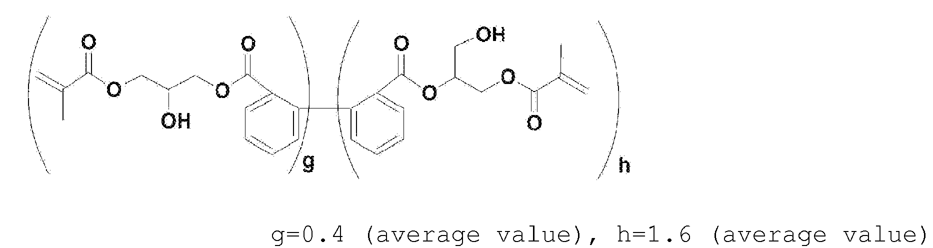

### 4-BPHMA :

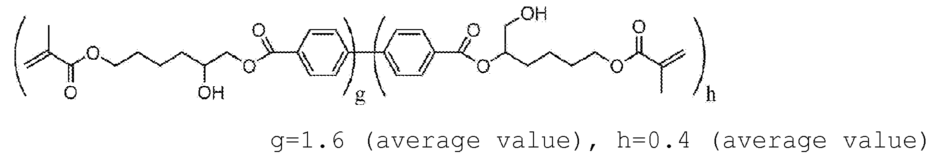

### 4-BPEPMA :

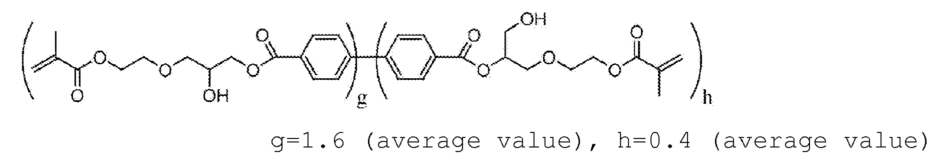

### 4-BPHMAI

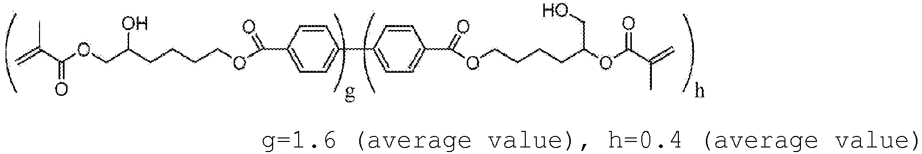

BGMA (for reference only, not according to the invention) shown below is a polymerizable monomer formed of three kinds of structural isomers (compounds represented by the following (a), (b), and (c)), and the ratio among (a), (b), and (c) means a molar ratio.

### BGMA :

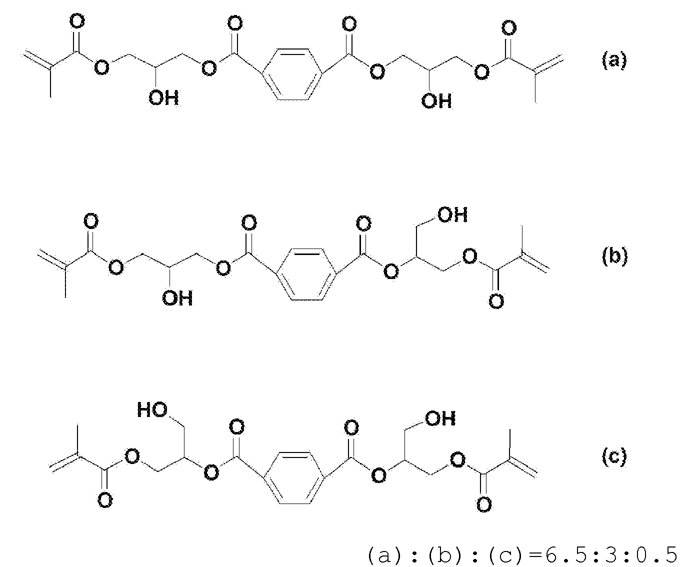

NGMA (for reference only, not according to the invention) shown below is a polymerizable monomer formed of three kinds of structural isomers (compounds represented by the following (a), (b), and (c)), and the ratio among (a), (b), and (c) means a molar ratio.

### NGMA :

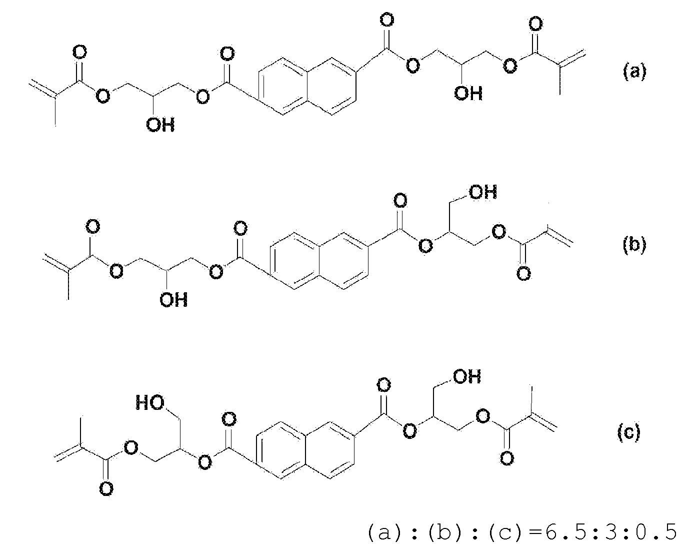

NGMAI (for reference only, not according to the invention) shown below is a polymerizable monomer formed of three kinds of structural isomers (compounds represented by the following (a), (b), and (c)), and the ratio among (a), (b), and (c) means a molar ratio.

### NGMAI :

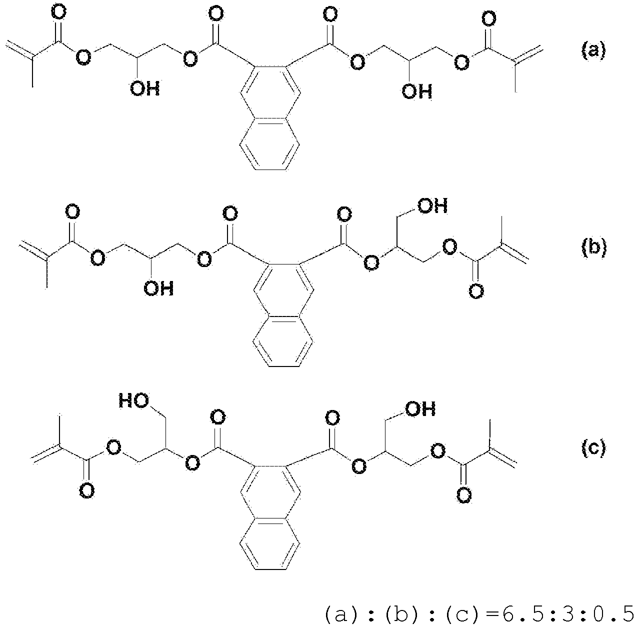

AGMA (for reference only, not according to the invention) shown below is a polymerizable monomer formed of three kinds of structural isomers (compounds represented by the following (a), (b), and (c)), and the ratio among (a), (b), and (c) means a molar ratio.

### AGMA :

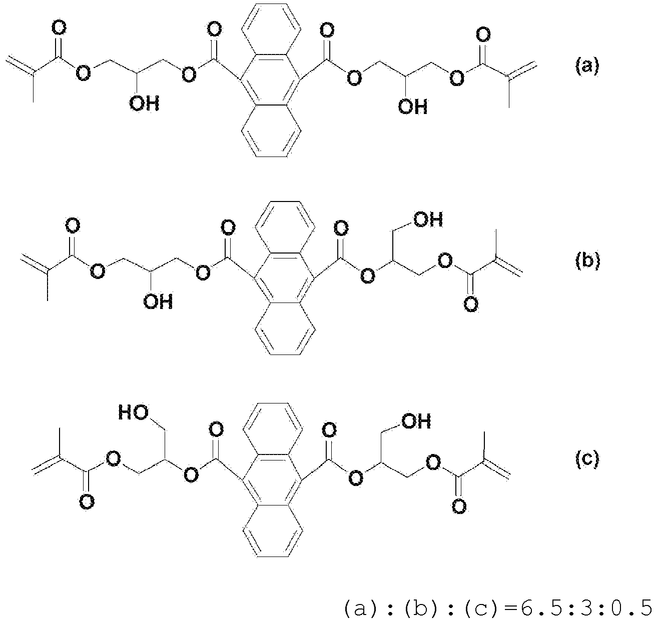

### [First Polymerizable Monomer (Polymerizable Monomer having Molecular Structure other than General Formula (1))]

### Bis-GMA :

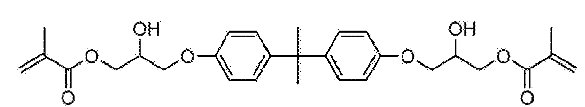

### 2-BPDGMA :

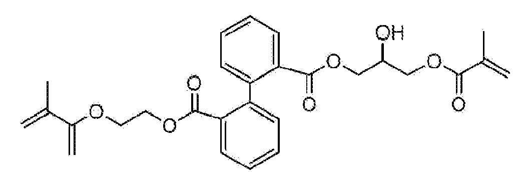

### 4-BPDM :

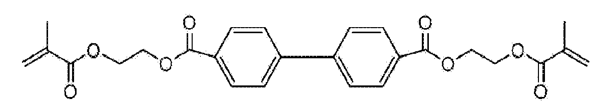

ErMA (molecular weight: 360): pentaerythritol dimethacrylate

### [Second Polymerizable Monomer]

3G: triethylene glycol dimethacrylate
ND: 1,9-nonanediol dimethacrylate
TCD: tricyclodecane dimethanol dimethacrylate
PM: mixture obtained by mixing 2-methacryloyloxyethyl dihydrogen phosphate and bis(2-methacryloyloxyethyl) hydrogen phosphate at a molar ratio of 1:1
HEMA: 2-hydroxyethyl methacrylate

### [Photopolymerization Initiator]

CQ: camphorquinone
DMBE: ethyl p-N,N-dimethylaminobenzoate

### [Polymerization Inhibitor]

BHT: 2,6-di-t-butyl-p-cresol

### [Inorganic Filler]

F1: mixture obtained by mixing spherical silica-zirconia (average particle diameter: 0.4 µm) subjected to hydrophobizing treatment with γ-methacryloyloxypropyltrimethoxysilane, and spherical silica-zirconia (average particle diameter: 0.07 µm) subjected to hydrophobizing treatment with γ-methacryloyloxypropyltrimethoxysilane at a mass ratio of 70:30.

### (2) Viscosity Measurement

The viscosity of a polymerizable monomer was measured using a CS rheometer. A measuring apparatus used was a viscoelasticity measuring apparatus CS rheometer "CVO120HR" (manufactured by Bohlin Instruments Ltd.) including a 4 cm/2° cone/plate geometry and a temperature control system. In addition, measurement was performed three times under the measurement conditions of a measurement temperature (plate temperature) of 25°C and a shear rate of 1 rps, and the average value of the measured values of the three times was defined as a viscosity.

### (3) Preparation of Matrix Monomer Sample

A first polymerizable monomer and a second polymerizable monomer were mixed at a predetermined mass ratio shown in Table 1. With respect to 100 parts by mass of the mixture of those polymerizable monomers, 0.5 part by mass of CQ, 0.8 part by mass of DMBE, and 0.1 part by mass of BHT were added, and then the mixture was stirred in the dark until becoming homogeneous. Thus, a matrix monomer sample was obtained.

### (4A) Preparation of Curable Composition Sample for Hardness and Bending Strength Evaluation

As shown in Table 1, 101.4 parts by mass of the above-mentioned matrix monomer sample and 513 parts by mass of the inorganic filler F1 were weighed out, and mixed in an agate mortar to provide a mixture . Subsequently, the mixture was degassed under vacuum to remove air bubbles, to thereby provide a paste-like curable composition sample for hardness and bending strength evaluation. The inorganic filler-containing curable composition is one that may be utilized as a dental curable composition, preferably a composite resin. As shown in Table 1, some matrix monomer samples were also degassed under vacuum to remove air bubbles without being mixed with the inorganic filler, to thereby prepare curable composition samples for bending strength evaluation.

In the preparation of the curable composition sample, in the case of using Bis-GMA as the first polymerizable monomer, at the time of weighing, Bis-GMA was weighed in a state of being heated to 60°C in an incubator. In addition, in the case of using 4-BPDM as the first polymerizable monomer, when 4-BPDM and the second polymerizable monomer were mixed, the mixing was performed while heating was performed with a dryer. Meanwhile, when the curable composition sample was prepared using the polymerizable monomer represented by the general formula (1) or ErMA as the first polymerizable monomer, the entire process of the preparation work was performed under a room temperature environment (25°C).

### (4B) Measurement of Bending Strength of Cured Product

In accordance with a bending strength measurement method for a dental filling composite resin of Class 2, Group 1 specified by JIS T6514:2013, each sample was subjected to visible light irradiation with a visible light irradiator (TOKUSO POWER LITE manufactured by Tokuyama Corporation) for 20 seconds to prepare a cured product sample, and its bending strength was measured. A universal testing machine (Autograph manufactured by Shimadzu Corporation) was used for the measurement.

The measurement of bending strength was performed for each of: a cured product sample (initial) obtained by curing the curable composition sample immediately after preparation; and a cured product sample (after storage at 60°C) obtained by curing the prepared curable composition sample after storage under an environment of 60°C in an incubator for 5 months. The measurement of the bending strength after storage at 60°C was performed for only some kinds of cured product samples.

### (4C) Measurement of Hardness of Paste (Inorganic Filler-containing Curable Composition Sample)

In a thermostatic chamber at 23°C, a paste (inorganic filler-containing curable composition sample) was filled into a nut-shaped mold made of SUS, and its surface was leveled off. The resultant was left to stand for 2 minutes. A ϕ 5 mm bar made of SUS was mounted as a pressure-sensitive shaft on a Sun rheometer (manufactured by Sun Scientific Co., Ltd.), and was compressed to enter at a rate of 60 mm/minute to a depth of 1 mm, or at a rate of 120 mm/minute to a depth of 2 mm. The maximum load [kg] at this time was defined as the hardness of the paste.

### (5A) Preparation of Curable Composition Sample for Adhesive Strength Test

A first polymerizable monomer and a second polymerizable monomer were mixed at a predetermined mass ratio shown in Table 2. Next, with respect to 100 parts by mass of the mixture of those polymerizable monomers, 1.5 parts by mass of CQ, 1.5 parts by mass of DMBE, 10 parts by mass of purified water, 85 parts by mass of acetone, and 0.3 part by mass of BHT were added, and then the mixture was stirred in the dark until becoming homogeneous . Thus, a curable composition sample for an adhesive strength test was obtained. The curable composition sample is one that may be utilized as a dental adhesive (so-called bonding material).

### (5B) Adhesive Strength Test Methods

As an adhesive strength test, evaluation was performed using a member having a surface showing hydrophilicity as a bonding obj ect. In this test, as the member having a surface showing hydrophilicity, a tooth having dentin exposed to its surface was selected. The dentin is a hydrophilic material containing hydroxyapatite, water, and other organic substances. Details of adhesive strength test methods are described below.

### (5B-1) Initial Adhesive Strength

A bovine front tooth extracted within 24 hours after killing was polished with waterproof abrasive paper P600 under a flow of water to carve out a dentin plane so as to be parallel to a labial surface and flat. Next, the carved-out plane was dried by being blown with compressed air for about 10 seconds. Next, a double-sided tape having a hole having a diameter of 3 mm was bonded onto the plane, and further, a paraffin wax having a thickness of 0.5 mm and having a hole having a diameter of 8 mm was fixed with the center of the hole of the paraffin wax arranged on the center of the hole of the previously bonded double-sided tape, to thereby form a simulated cavity. To the simulated cavity, the curable composition sample for an adhesive strength test (curable composition sample immediately after preparation) was applied, left to stand for 20 seconds, and then dried by being blown with compressed air for about 10 seconds. Visible light irradiation was performed for 10 seconds with a visible light irradiator (TOKUSO POWER LITE manufactured by Tokuyama Corporation) to cure the curable composition sample. Further, a composite resin (ESTELITE SIGMA QUICK manufactured by Tokuyama Dental Corporation) was filled thereon, and the resultant was pressed with a polyester sheet. After the filling, the resultant was similarly cured by being irradiated with visible light for 10 seconds to prepare a bonded test piece.

Onto the composite resin cured body top surface of the bonded test piece, resin cement (BISTITE II manufactured by Tokuyama Dental Corporation) was applied, and an attachment made of SUS having a cylindrical shape having a diameter of 8 mm and a length of 25 mm was further bonded. Next, the resin cement was cured at 37°C for 15 minutes, and then the bonded test piece was immersed in water at 37°C for 24 hours, to thereby provide a bonded test piece I with an attachment. After that, the bonded test piece I with an attachment was pulled using a universal testing machine (Autograph manufactured by Shimadzu Corporation) at a crosshead speed of 1 mm/min, and tensile adhesive strength between the tooth and the composite resin cured body was measured. The tensile adhesive strength was measured for each of eight test pieces prepared for each Example or Comparative Example. Then, the average value of the tensile adhesive strengths of the eight times was defined as the initial adhesive strength of each Example or Comparative Example.

### (5B-2) Adhesive Strength after Endurance Test

The bonded test piece I with an attachment was put into a thermal shock tester, and subjected to an endurance test involving repeating the following operations 3,000 times: the test piece was immersed in a water bath at 4°C for 1 minute, then transferred to a water bath at 60°C and immersed therein for 1 minute, and returned to the water bath at 4°C again. After that, for the bonded test piece I subjected to the endurance test, tensile adhesive strength was measured in the same manner as in the case of determining the initial adhesive strength, and was defined as adhesive strength after an endurance test.

### (5B-3) Adhesive Strength after Storage at 60°C

Adhesive strength was measured by the procedure described in (5B-1) except that the prepared curable composition sample that had been stored in an incubator under an environment of 60°C for 20 days was used inplace of the curable composition sample immediately after preparation used in the measurement of the initial adhesive strength.

### (6) Synthesis Procedure for Polymerizable Monomer

Of the first polymerizable monomers used in the preparation of the matrix monomer samples, ones corresponding to the polymerizable monomer represented by the general formula (1) were synthesized by the following procedures.

### <Synthesis of 4-BPGMA>

To 12.8 g (0.09 mol) of glycidyl methacrylate, 12.1 g (0.05 mol) of 4,4'-biphenyldicarboxylic acid, 0.02 g (0.00009 mol) of benzyltriethylammonium chloride, 0.02 g (0.00009 mol) of BHT, and 20 g of dimethylformamide were added, and the mixture was subjected to a reaction at 100°C for 4 hours. To the resultant liquid, 40 ml of ethyl acetate was added, and the mixture was turned into a homogeneous solution. After that, the solution was transferred to a separatory funnel and washed three times with 40 ml of a 10 wt% aqueous solution of potassium carbonate. The resultant was further washed three times with distilled water, and then magnesium sulfate was added to the collected ethyl acetate layer to remove the contained water. After that, the magnesium sulfate was filtered off, and the filtrate was concentrated with a rotary evaporator. The concentrate was further vacuum-dried to provide 4-BPGMA (yield:22.3 g, percentage yield: 85%, HPLC purity: 95%). ¹H NMR spectrum data on the resultant 4-BPGMA was as follows.
¹H NMR δ 1.93 (s, 6H), 3.95 (d, 0.8H), 4.30-4.70 (m, 9.2H), 6.10 (s, 2H), 7.59 (d, 4H), 8.06 (s, 4H)

### <Synthesis of 4-BPGMAI>

A mixed liquid of 11.5 g (0.052 mol) of 4,4'-biphenyldicarboxylic acid, 0.46 g (0.0065 mol) of dimethylformamide, and 40 ml of toluene was prepared. To the mixed liquid in a stirred state, a mixed liquid of 24.8 g (0.208 mol) of thionyl chloride and 10 ml of toluene was slowly added dropwise under room temperature. The mixed liquid after the completion of the dropwise addition was heated to 95°C and refluxed for 3 h. The resultant yellow transparent liquid was allowed to cool, and thus a toluene solution of 4,4'-biphenyldicarboxylic acid chloride was obtained. The toluene solution was subjected to a rotary evaporator to remove toluene, thionyl chloride, and hydrogen chloride at 40°C, and thus 13.3 g (0.052 mol) of 4,4'-diphenyletherdicarboxylic acid chloride (hereinafter referred to as "acid chloride A") was obtained asasolid. After that, to the solid acid chloride A, 30 ml of methylene chloride was added to prepare an acid chloride A-methylene chloride solution.

Separately, a solution was prepared by mixing 33.3 g (0.208 mol) of glycerol-1-yl monomethacrylate, 12.1 g (0.104 mol) of tetramethylethylenediamine, 0.002 g of BHT and 20 ml of methylene chloride . The solution was slowly added dropwise to the acid chloride A-methylene chloride solution at -78°C, and the mixture was further stirred for 5 hours. The resultant liquid was washed three times with 60 ml of 0.4 mol/L aqueous hydrochloric acid, and further washed three times with 60 ml of a 10 wt% aqueous solution of potassium carbonate. The resultant was further washed three times with distilled water, and then magnesium sulfate was added to the collected methylene chloride layer to remove the contained water. After that, the magnesium sulfate was filtered off, and the filtrate was concentrated with a rotary evaporator. The concentrate was purified by silica gel column chromatography to provide 4-BPGMAI (yield: 25.2 g, percentage yield: 92%, HPLC purity: 97%) . ¹H NMR spectrum data on the resultant 4-BPGMAI was as follows.
¹H NMR δ 1.93 (s, 6H), 3.95 (d, 0.2H), 4.30-4.70 (m, 9.8H), 5.59 (s, 2H), 6.10 (s, 2H), 7.59 (d, 4H), 8.06 (s, 4H)

### <Synthesis of 4-BPGMAII>

4-BPGMA was synthesized by the above-mentioned method, and then purified by silica gel column chromatography (filler: SiO₂, developing solvent: ethyl acetate/hexane=3/1 to 2/1) to provide 4-BPGMAII (yield: 16.8 g, percentage yield: 64%, HPLC purity: 99%) . ¹H NMR spectrum data on the resultant 4-BPGMAII was as follows.
¹H NMR δ 1.93 (s, 6H), 4.30-4.45 (m, 10H), 5.59 (s, 2H), 6.10 (s, 2H), 7.59 (d, 4H), 8.06 (s, 4H)

### <Synthesis of 4-BPGMAIII>

4-BPGMA was synthesized by the above-mentioned method, and then purified by silica gel column chromatography (filler: SiO₂, developing solvent: ethyl acetate/hexane=3/1 to 2/1) to provide 4-BPGMAIII (yield: 2.1 g, percentage yield: 8%, HPLC purity: 97%) . ¹H NMR spectrum data on the resultant 4-BPGMAIII was as follows.
¹H NMR δ 1.93 (s, 6H), 3.95 (d, 3.2H), 4.30-4.70 (m, 6.8H), 5.59 (s, 2H), 6.10 (s, 2H), 7.59 (d, 4H), 8.06 (s, 4H)

### <Synthesis of 2-BPGMA>

2-BPGMA (yield: 22.6 g, percentage yield: 86%, HPLC purity: 96%) was obtained by the same method as the synthesis method for 4-BPGMA except that 12.1 g (0.05 mol) of 2,2'-biphenyldicarboxylic acid was used in place of 12.1 g (0.05 mol) of 4,4'-biphenyldicarboxylic acid. ¹H NMR spectrum data on the resultant 2-BPGMA was as follows.
¹H NMR δ 1.93 (s, 6H), 3.83 (d, 2H), 4.00-4.65 (m, 8H), 5.59 (s, 2H), 6.16 (s, 2H), 7.34 (d, 2H), 7.56 (d, 4H), 8.06 (d, 2H)

### <Synthesis of 2-BPGMAI>

43.5 g (0.135 mol) of diallyl 2,2'-biphenyldicarboxylate was dissolved in 50 ml of methylene chloride, and then 58 g of a 60 mass% m-chloroperbenzoic acid/water mixture (corresponding to 0.338 mol) was added, followed by stirring at room temperature for 10 hours. After the stirring, m-chlorobenzoic acid, a by-product, was filtered off, and the filtrate was subjected to reducing treatment with 75 ml of a 15 mass% aqueous solution of sodium sulfite. The methylene chloride layer was subjected to liquid separation, and then washed twice with a 5 mass% aqueous solution of potassium carbonate. The resultant was dried over magnesium sulfate, and then concentrated with a rotary evaporator. The concentrate was further vacuum-dried to provide 44.0 g of 2,2'-biphenyldicarboxy glycidyl ester (percentage yield: 92%).

22.1 g (0.0625 mol) of the resultant 2,2'-biphenyldicarboxy glycidyl ester, 13.4 g (0.156 mol) of methacrylic acid, 0.045 g (0.2 mmol) of benzyltriethylammonium chloride, and 0.03 g of p-methoxyphenol were stirred at 90°C for 4 hours. After the heating and stirring, the resultant was allowed to cool to room temperature, and 50 ml of water was added, followed by extraction with methylene chloride. The resultant methylene chloride layer was dried over magnesium sulfate, and then concentrated with a rotary evaporator. The concentrate was further vacuum-dried to provide 2-BPGMAI (yield: 24.5 g, percentage yield: 75%, HPLCpurity: 95%) . ¹H NMR spectrum data on the resultant 2-BPGMAI was as follows.
¹H NMR δ 1.93 (s, 6H), 3.81 (d, 2H), 4.00-4.65 (m, 8H), 5.58 (s, 2H), 6.15 (s, 2H), 7.33 (d, 2H), 7.57 (d, 4H), 8.09 (d, 2H)

### <Synthesis of 2-BPGMAII>

2-BPGMAII (yield: 22.6 g, percentage yield: 93%, HPLC purity: 96%) was obtained by the same method as the synthesis method for 4-BPGMAI except that 12.1 g (0.05mol) of 2,2'-biphenyldicarboxylic acid was used in place of 12.1 g (0.05 mol) of 4,4'-biphenyldicarboxylic acid. ¹H NMR spectrum data on the resultant 2-BPGMAII was as follows.
¹H NMR δ 1.93 (s, 6H), 3.83 (d, 0.2H), 4.00-4.65 (m, 9.8H), 5.59 (s, 2H), 6.16 (s, 2H), 7.34 (d, 2H), 7.56 (d, 4H), 8.06 (d, 2H)

### <Synthesis of 2-BPGMAIII>

2-BPGMA was synthesized by the above-mentioned method, and then purified by silica gel column chromatography (filler: SiO₂, developing solvent: ethyl acetate/hexane=3/1 to 2/1) to provide 2-BPGMAIII (yield: 17.6 g, percentage yield: 67%, HPLC purity: 99%) . ¹H NMR spectrum data on the resultant 2-BPGMAIII was as follows.
¹H NMR δ 1.93 (s, 6H), 4.30-4.41 (m, 10H), 5.59 (s, 2H), 6.16 (s, 2H), 7.34 (d, 2H), 7.56 (d, 4H), 8.06 (d, 2H)

### <Synthesis of 2-BPGMAIV>

4-BPGMA was synthesized by the above-mentioned method, and then purified by silica gel column chromatography (filler: SiO₂, developing solvent: ethyl acetate/hexane=3/1 to 2/1) to provide 2-BPGMAIV (yield: 2.2 g, percentage yield: 9%, HPLC purity: 96%) . ¹H NMR spectrum data on the resultant 2-BPGMAIV was as follows.
¹H NMR δ 1.93 (s, 6H), 3.83 (d, 3.2H), 4.00-4.65 (m, 6.8H), 5.59 (s, 2H), 6.16 (s, 2H), 7.34 (d, 2H), 7.56 (d, 4H), 8.06 (d, 2H)

### <4-BPHMA>

5-Hexen-1-ol (30.1 g, 0.3 mol) was dissolved in 100 ml of methylene chloride, and then 33.4 g (0.33 mol) of triethylamine and 1.8 g (0.015 mol) of N,N-dimethylaminopyridine were added. The resultant solution was cooled with ice, and a methylene chloride (50 ml) solution of 31.4 g (0.3 mol) of methacrylic acid chloride was added dropwise. The solution after the completion of the dropwise addition was stirred at room temperature for 3 hours, and then 100 ml of distilled water was added, followed by three times of extraction with methylene chloride. The resultant methylene chloride layer was subjected to solvent removal with a rotary evaporator, and then the residue was dissolved in 100 ml of toluene. The resultant toluene solution was washed three times with a 0.5 N hydrochloric acid solution, and then washed three times with brine, followed by drying over magnesium sulfate solution. After the drying, the magnesium sulfate solution was filtered off, and the filtrate was concentrated with a rotary evaporator. The concentrate was further vacuum-dried to provide 46.4 g of 5-hexen-1-yl methacrylate (percentage yield: 92%) .

45.4 g (0.27 mol) of the resultant 5-hexen-1-yl methacrylate was dissolved in 100 ml of methylene chloride, and then 196 g of a 60 mass% m-chloroperbenzoic acid/water mixture (corresponding to 0.675 mol) was added, followed by stirring at room temperature for 10 hours. After the stirring, m-chlorobenzoic acid, a by-product, was filtered off, and the filtrate was subjected to reducing treatment with 150 ml of a 15 mass% aqueous solution of sodium sulfite. The methylene chloride layer was subjected to liquid separation, and then washed twice with a 5 mass% aqueous solution of potassium carbonate. The resultant was dried over magnesium sulfate, and then concentrated with a rotary evaporator. The concentrate was further vacuum-dried to provide 44.8 g of 5,6-epoxyhexan-1-yl methacrylate (percentage yield: 90%).

A mixed liquid obtained by mixing 23.0 g (0.125 mol) of the resultant 5,6-epoxyhexan-1-yl methacrylate, 12.1 g (0.05 mol) of 4,4'-biphenyldicarboxylic acid, 0.045 g (0.2 mmol) of benzyltriethylammonium chloride, and 0.03 g of p-methoxyphenol was stirred at 90°C for 4 hours. After the heating and stirring, the resultant was allowed to cool to room temperature, and 50 ml of water was added, followed by extraction with methylene chloride. The resultant methylene chloride layer was dried over magnesium sulfate, and then concentrated with a rotary evaporator. The concentrate was further vacuum-dried to provide 4-BPHMA (yield: 28.1 g, 0.046 mol, percentage yield: 93%, HPLC purity: 95%). ¹H NMR spectrum data on the resultant 4-BPHMA was as follows.
¹H NMR δ 1.29 (t, 4H), 1.44 (m, 4H), 1.57 (t, 4H), 1.93 (s, 6H), 3.85 (d, 1.9H), 4.15-4.70 (m, 8.1H), 5.59 (s, 2H), 6.16 (s, 2H), 7.57 (d, 4H), 8.05 (d, 4H)

### <4-BPEPMA>

30.6 g (0.3 mol) of allyloxyethanol was dissolved in 100 ml of methylene chloride, and then 33.4 g (0.33 mol) of triethylamine and 1.8 g (0.015 mol) of N,N-dimethylaminopyridine were added. The resultant solution was cooled with ice, and 50 ml of a methylene chloride solution of 31.4 g (0.3 mol) of methacrylic acid chloride was added dropwise. The solution after the completion of the dropwise addition was stirred at room temperature for 3 hours, and then 100 ml of distilled water was added, followed by three times of extraction with methylene chloride. The resultant methylene chloride layer was subjected to solvent removal using a rotary evaporator, and then the residue was dissolved in 100 ml of toluene. The resultant toluene solution was washed three times with a 0.5 N hydrochloric acid solution, and then washed three times with brine, followed by drying over magnesium sulfate solution. After the drying, the magnesium sulfate solution was filtered off, and the filtrate was concentrated with a rotary evaporator. The concentrate was further vacuum-dried to provide 46.0 g of allyloxyethyl methacrylate (percentage yield: 90%).

46.0 g (0.27 mol) of the resultant allyloxyethyl methacrylate was dissolved in 100 ml of methylene chloride, and then 196 g of a 60 mass% m-chloroperbenzoic acid/water mixture (corresponding to 0.675 mol) was added, followed by stirring at room temperature for 10 hours. After the stirring, m-chlorobenzoic acid, a by-product, was filtered off, and the filtrate was subjected to reducing treatment with 150 ml of a 15 mass% aqueous solution of sodium sulfite. The methylene chloride layer was subjected to liquid separation, and then washed twice with a 5 mass% aqueous solution of potassium carbonate. The resultant was dried over magnesium sulfate, and then concentrated with a rotary evaporator. The concentrate was further vacuum-dried to provide 47.3 g of glycidyloxyethyl methacrylate (percentage yield: 94%).

A mixed liquid obtained by mixing 23.3 g (0.125 mol) of the resultant glycidyloxyethyl methacrylate, 12.1 g (0.05 mol) of 4,4'-biphenyldicarboxylic acid, 0.045 g (0.2 mmol) of benzyltriethylammonium chloride, and 0.03 g of p-methoxyphenol was stirred at 90°C for 4 hours. After the heating and stirring, the resultant was allowed to cool to room temperature, and 50 ml of water was added, followed by extraction with methylene chloride. The resultant methylene chloride layer was dried over magnesium sulfate, and then concentrated with a rotary evaporator. The concentrate was further vacuum-dried to provide 4-BPEPMA (yield: 28.3 g, percentage yield: 92%, HPLC purity: 94%) . ¹H NMR spectrum data on the resultant 4-BPEPMA was as follows.
¹H NMR δ 1.93 (s, 6H), 3.50-4.50 (m, 18H), 4.15-4.70 (m, 8.1H), 5.59 (s, 2H), 6.16 (s, 2H), 7.58 (d, 4H), 8.03 (d, 4H)

### <4-BPHMAI>

A mixed liquid obtained by mixing 24.1 g (0.10 mol) of 4,4'-biphenyldicarboxylic acid, 5-hexen-1-ol (21.0 g, 0.21 mol), and p-toluenesulfonic acid (0.01 mol, 1.72 g) was heated and stirred at 120°C for 2 hours, and then water produced as a by-product was removed under reduced pressure. The resultant was allowed to cool to room temperature. After that, a 5 mass% aqueous solution of potassium carbonate was added, and the organic layer was extracted with toluene. The resultant toluene layer was further washed twice with brine, and dried over magnesium carbonate. After the drying, the toluene was removed by evaporation using a rotary evaporator to provide bis(5-hexene)-4,4'-biphenyldicarboxylate (yield: 38.6 g, percentage yield: 95%).

32.5 g (0.08 mol) of the resultant bis(5-hexene)-4,4'-biphenyldicarboxylate was dissolved in 100 ml of methylene chloride, and then 34.5 g of a 60 mass% m-chloroperbenzoic acid/water mixture (corresponding to 0.12 mol) was added, followed by stirring at room temperature for 10 hours. After the stirring, m-chlorobenzoic acid, a by-product, was filtered off, and the filtrate was subjected to reducing treatment with 150 ml of a 15 mass% aqueous solution of sodium sulfite. The methylene chloride layer was subjected to liquid separation, and then washed twice with a 5 mass% aqueous solution of potassium carbonate . The resultant was dried over magnesium sulfate, and then concentrated with a rotary evaporator. The concentrate was further vacuum-dried to provide 31.6 g of bis(5,6-epoxyhexan-1-yl) 4,4'-biphenyldicarboxylate (percentage yield: 90%).

A mixed liquid obtained by mixing 27.4 g (0.0625 mol) of the resultant bis(5,6-epoxyhexan-1-yl) 4,4'-biphenyldicarboxylate, 13.4 g (0.156 mol) of methacrylic acid, 0.045 g (0.2 mmol) of benzyltriethylammonium chloride, and 0.03 g of p-methoxyphenol was stirred at 90°C for 4 hours. After the heating and stirring, the mixed liquid was allowed to cool to room temperature, and 50 ml of water was added, followed by extraction with methylene chloride. The resultant methylene chloride layer was dried over magnesium sulfate, and then concentrated with a rotary evaporator. The concentrate was further vacuum-dried to provide 4-BPHMAI (yield: 28.6 g, percentage yield: 75%, HPLC purity: 96%) . ¹H NMR spectrum data on the resultant 4-BPHMAI was as follows.
¹H NMR δ 1.23-1.76 (m, 6H), 1.93 (d, 6H), 3.80 (m, 4H), 4.03 (m, 1.6H), 4.24 (m, 4H), 4.31 (d, 2.4H), 5.58 (s, 2H), 6.15 (s, 2H), 7.58 (d, 2H), 8.04 (d, 4H),

### <Synthesis of BGMA (for reference only, not according to the invention)>

BGMA (yield: 20.7 g, percentage yield: 92%, HPLC purity: 95%) was obtained by the same method as the synthesis method for 4-BPGMA except that 8.3 g (0.05 mol) of terephthalic acid was used in place of 12.1 g (0.05 mol) of 4,4'-biphenyldicarboxylic acid. ¹H NMR spectrum data on the resultant BGMA was as follows.
¹H NMR δ 1.93 (s, 6H), 3.75 (d, 2H), 4.35-4.75 (m, 8H), 5.57 (s, 2H), 6.13 (s, 2H), 8.09 (d, 4H)

### <Synthesis of NGMA (for reference only, not according to the invention)>

NGMA (yield: 22.8 g, percentage yield: 91%, HPLC purity: 96%) was obtained by the same method as the synthesis method for 4-BPGMA except that 10.8 g (0.05 mol) of 2,6-naphthalenedicarboxylic acid was used in place of 12.1 g (0.05 mol) of 4,4'-biphenyldicarboxylic acid. ¹H NMR spectrum data on the resultant NGMA was as follows.
¹H NMR δ 1.93 (s, 6H), 3.85 (d, 2H), 4.40-4.75 (m, 8H), 5.58 (s, 2H), 6.15 (s, 2H), 7.90 (d, 2H), 8.09 (d, 2H), 8.57 (d, 2H)

### <Synthesis of NGMAI (for reference only, not according to the invention)>

40.0 g (0.135 mol) of diallyl 2,3-naphthalenedicarboxylate was dissolved in 50 ml of methylene chloride, and then 99 g of a 60 mass% m-chloroperbenzoic acid/water mixture (corresponding to 0.338 mol) was added, followed by stirring at room temperature for 10 hours. After the stirring, m-chlorobenzoic acid, a by-product, was filtered off, and the filtrate was subjected to reducing treatment with 75 ml of a 15 mass% aqueous solution of sodium sulfite. The methylene chloride layer was subjected to liquid separation, and then washed twice with a 1 N sodium hydroxide solution. The resultant was dried over magnesium sulfate, and then concentrated with a rotary evaporator. The concentrate was further vacuum-dried to provide 39.9 g of 2,3-naphthalenedicarboxylic acid diglycidyl ester (percentage yield: 90%).

A mixed liquid obtained by mixing 20.5 g (0.0625 mol) of the resultant 2,3-naphthalenedicarboxylic acid diglycidyl ester, 13.4 g (0.165 mol) of methacrylic acid, 0.045 g (0.2 mmol) of benzyltriethylammonium chloride, and 0.03 g of p-methoxyphenol was stirred at 90°C for 4 hours. After the heating and stirring, the resultant was allowed to cool to room temperature, and 50 ml of water was added, followed by extraction with methylene chloride. The resultant methylene chloride layer was dried over magnesium sulfate, and then concentrated with a rotary evaporator. The concentrate was further vacuum-dried to provide NGMAI (yield: 22.5 g, percentage yield: 72%, HPLC purity: 95%) . ¹H NMR spectrum data on the resultant NGMAI was as follows.
¹H NMR δ 1.93 (s, 6H), 3.84 (d, 2H), 4.40-4.75 (m, 8H), 5.58 (s, 2H), 6.15 (s, 2H), 7.60 (d, 2H), 7.94 (d, 2H), 8.60 (d, 2H)

### <Synthesis of AGMA (for reference only, not according to the invention)>

AGMA (yield: 24.8 g, percentage yield: 90%, HPLC purity: 94%) was obtained by the same method as the synthesis method for 4-BPGMA except that 13.3 g (0.05 mol) of 9,10-anthracenedicarboxylic acid was used in place of 12.1 g (0.05 mol) of 4,4'-biphenyldicarboxylic acid. ¹H NMR spectrum data on the resultant AGMA was as follows.
¹H NMR δ 1.93 (s, 6H), 3.80 (d, 2H), 4.42-4.70 (m, 8H), 5.55 (s, 2H), 6.12 (s, 2H), 7.55 (d, 4H), 8.62 (d, 4H)

### (7) Evaluation Results

The compositions of the curable composition samples prepared by using the first polymerizable monomers and the evaluation results of the hardnesses and bending strengths of their cured products are shown in Table 1, and the compositions of the curable composition samples prepared using the first polymerizable monomers and the evaluation results of their adhesive strengths are shown in Table 2.

**Table 1**

| | Composition of curable composition sample (part(s) by mass) | | | | | Hardness of paste/kg | Cured product | |
|---|---|---|---|---|---|---|---|---|
| | First polymerizable monomer (60) | | Second polymerizable monomer (40) | Other components, such as polymerization initiator | Filler | | Bending strength/MPa (deviation) | |
| | Abbreviation | Viscosity /Pa·s | | | | | Initial | After storage at 60°C |
| Example A1 | 4-BPGMA | 36 | 3G | | - | - | 63 (2.1) | - |
| Example A2 | | | | | | 3.8 | 153 (5.1) | 142 (6.2) |
| Example A3 | | | ND | | | 3.9 | 151 (4.3) | - |
| Example A4 | | | TCD | | | 4.0 | 144 (3.8) | - |
| Example A5 | 4-BPGMAI | 34 | | | | 3.5 | 155 (4.2) | - |
| Example A6 | 4-BPGMAII | 34 | | | | 3.5 | 158 (3.5) | 112 (2.6) |
| Example A7 | 4-BPGMAIII | 37 | | | | 3.5 | 150 (3.0) | 146 (1.8) |
| Example A8 | 2-BPGMA | 34 | | | | 3.4 | 152 (4.3) | 144 (7.0) |
| Example A9 | 2-BPGMAI | 35 | | | | 3.4 | 151 (4.8) | - |
| Example A10 | 2-BPGMAII | 33 | | | | 3.4 | 154 (4.0) | 137 (4.2) |
| Example A11 | 2-BPGMAIII | 33 | | | | 3.5 | 157 (3.2) | 119 (4.2) |
| Example A12 | 2-BPGMAIV | 34 | | | | 3.4 | 149 (2.2) | 146 (2.9) |
| Example A13 | 4-BPHMA | 32 | | | | 3.2 | 141 (3.9) | - |
| Example A14 | 4-BPEPMA | 31 | | | F1 | 3.0 | 140 (2.9) | - |
| Example A15 | 4-BPHMAI | 35 | | | (513) | 3.9 | 140 (6.1) | - |
| Example A16 (for reference only) | BGMA | 21 | | CQ (0.5)/DMBE (0.8)/BHT (0.1) | | 2.4 | 135 (3.4) | - |
| Example A17 (for reference only) | NGMA | 25 | 3G | | | 2.5 | 132 (3.1) | - |
| Example A18 (for reference only) | NGMAI | 26 | | | | 2.5 | 133 (2.7) | - |
| Example A19 (for reference only) | AGMA | 27 | | | | 3.1 | 130 (3.8) | - |
| Comparative Example A1 | Bis-GMA | >100 | | | - | - | 60 (2.3) | - |
| Comparative Example A2 | Bis-GMA | >100 | | | F1 (513) | 5.0 | 138 (7.2) | - |
| Comparative Example A3 | 4-BPDM | Unmeasurable (solid) | | | | 4.6 | 115 (5.8) | - |
| Comparative Example A4 | 2-BPDGMA | 32 | | | | 4.0 | 119 (4.2) | - |
| Comparative Example A5 | ErMA | 12 | | | | 1.2 | 89 (3.1) | - |

**Table 2**

| | Composition of curable composition sample (part(s) by mass) | | | Adhesive strength/Mpa (deviation) | | |
|---|---|---|---|---|---|---|
| | First polymerizable monomer (30) | Second polymerizable monomer | Other components, such as polymerization initiator | | | |
| | | | | Initial | After endurance test | After storage at 60°C |
| Example B1 | 4-BPGMA | 3G (20)/PM (30)/HEMA (20) | CQ (1.5)/DMBE (1.5)/BHT (0.3)/Water (10)/Acetone (85) | 12.9 (1.9) | 11.5 (1.5) | 10.3 (1.2) |
| Example B2 | 4-BPGMAI | | | 13.0 (2.2) | 12.0 (3.1) | 9.8 (1.7) |
| Example B3 | 4-BPGMAII | | | 13.3 (2.7) | 12.1 (1.6) | 6.9 (1.8) |
| Example B4 | 4-BPGMAIII | | | 12.3 (1.9) | 11.5 (1.2) | 11.0 (2.2) |
| Example B5 | 2-BPGMA | | | 12.7 (1.8) | 11.4 (1.6) | 10.5 (1.2) |
| Example B6 | 2-BPGMAII | | | 12.9 (2.2) | 11.8 (1.9) | 9.5 (1.4) |
| Example B7 | 2-BPGMAIII | | | 13.2 (1.1) | 12.0 (2.8) | 6.5 (1.3) |
| Example B8 | 2-BPGMAIV | | | 12.2 (1.3) | 11.9 (1.4) | 11.9 (2.1) |
| Example B9 (for reference only) | BGMA | | | 9.1 (0.8) | 7.4 (0.6) | - |
| Example B10 (for reference only) | NGMA | | | 9.8 (1.1) | 8.2 (0.9) | - |
| Example B11 (for reference only) | NGMAI | | | 9.7 (1.0) | 8.2 (0.8) | - |
| Example B12 (for reference only) | AGMA | | | 9.3 (1.5) | 8.0 (0.8) | - |
| Comparative Example B1 | Bis-GMA | | | 5.8 (1.0) | 4.8 (0.7) | - |
| Comparative Example B2 | 4-BPDM | | | 3.5 (0.3) | 2.9 (0.4) | - |
| Comparative Example B3 | 2-BPDGMA | | | 5.3 (1.5) | 3.8 (1.1) | - |
| Comparative Example B4 | ErMA | | | 9.1 (1.0) | 3.3 (1.3) | - |

## Claims

1. A polymerizable monomer, which is represented by the following general formula (1): 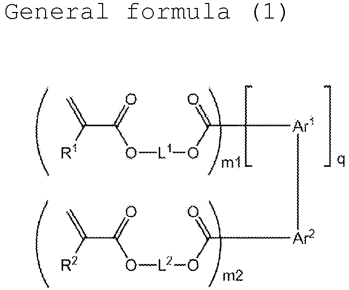 in the general formula (1), Ar¹ and Ar² each represent a substituted or unsubstituted aromatic group having a valence selected from divalence or trivalence, and may be identical to or different from each other, L¹ and L² each represent a divalent substituted hydrocarbon group having a main chain with a number of atoms within a range of from 2 to 10 and containing at least one hydroxy group, and may be identical to or different from each other, R¹ and R² each represent hydrogen or a methyl group, q is 1, and m1 and m2 each represent 1 or 2;
provided that the polymerizable monomer is other than:

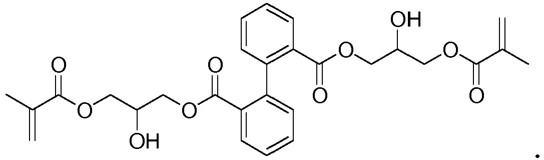

2. A polymerizable monomer according to claim 1, wherein a substituent of the substituted aromatic group is selected from a group consisting of a monovalent hydrocarbon group having 1 to 20 carbon atoms, -COOR³, -OR³, a halogen group, an amino group, a nitro group, and a carboxyl group, and R³ is a monovalent hydrocarbon group having 1 to 20 carbon atoms.

3. A polymerizable monomer according to claim 1 or 2, wherein the carbon atoms constituting the main chain of the hydrocarbon group may be substituted by a heteroatom, preferably wherein the heteroatom is an oxygen atom, a nitrogen atom, a sulfur atom, or a silicon atom.

4. A polymerizable monomer according to any of claims 1 to 3, wherein the atoms constituting the main chain of the hydrocarbon group may have bonded thereto a substituent, preferably wherein the substituent is an alkyl group having 1 to 3 carbon atoms, a halogen, -COOR⁴, or -OR⁴, wherein R⁴ is the same as the alkyl group having 1 to 3 carbon atoms.

5. A polymerizable monomer according to any of claims 1 to 4, wherein the polymerizable monomer is represented by the following general formula (2): 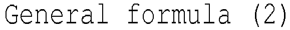 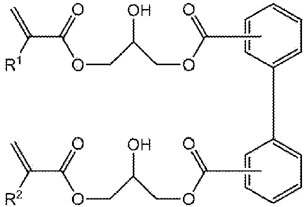 in the general formula (2), R¹ and R² are the same as those shown in the general formula (1).

6. A polymerizable monomer according to any of claims 1 to 4, wherein the polymerizable monomer is represented by the following general formula (3): 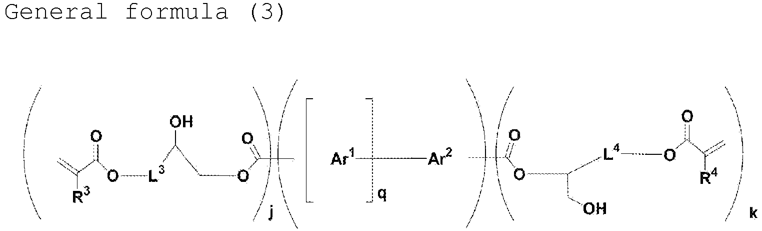 in the general formula (3), Ar¹ and Ar² are the same as those shown in the general formula (1) except that the valence may be only divalence, q is the same as that shown in the general formula (1), L³ and L⁴ each represent a divalent hydrocarbon group having a main chain with a number of atoms within a range of from 1 to 8, and may be identical to or different from each other, R³ and R⁴ each represent hydrogen or a methyl group, j represents 0, 1, or 2, k represents 0, 1, or 2, and j+k=2.

7. A polymerizable monomer according to claim 6, wherein a combination (j, k) of values j and k shown in the general formula (3) comprises a combination selected from the group consisting of (1, 1) and (0, 2).

8. A polymerizable monomer according to claim 6, wherein the polymerizable monomer comprises two or more kinds of structural isomers in each of which a combination (j, k) of values j and k shown in the general formula (3) is selected from the group consisting of (2, 0), (1, 1), and (0, 2).

9. A polymerizable monomer according to claim 8, wherein an average value of values k in all molecules of the polymerizable monomer is 0.05 or more and less than 2.0.

10. A method of producing a polymerizable monomer, the polymerizable monomer comprising two or more kinds of structural isomers selected from the group consisting of compounds represented by the following general formulae (6) to (8), the method comprising at least a reaction step of allowing a compound represented by the following general formula (4) and a compound represented by the following general formula (5) to react with each other: 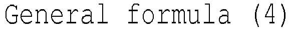 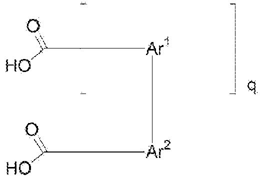 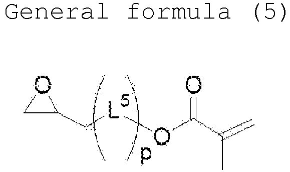 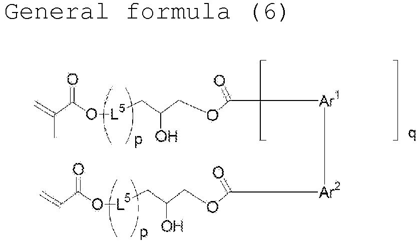 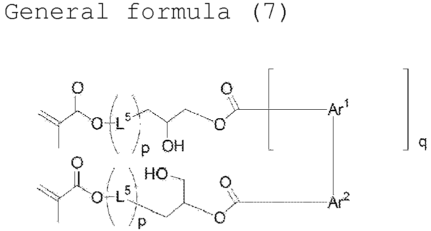 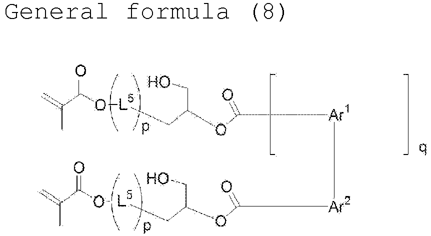 in the general formulae (4) to (8), Ar¹ and Ar² each represent a divalent substituted or unsubstituted aromatic group, and may be identical to or different from each other, L⁵ represents a divalent substituted or unsubstituted hydrocarbon group having a main chain with a number of atoms of from 1 to 7, p represents 0 or 1, and q is 1.

11. A method of producing a polymerizable monomer according to claim 10, wherein a substituent of the substituted aromatic group is selected from a group consisting of a monovalent hydrocarbon group having 1 to 20 carbon atoms, -COOR³, -OR³, a halogen group, an amino group, a nitro group, and a carboxyl group, and R³ is a monovalent hydrocarbon group having 1 to 20 carbon atoms.

12. A method of producing a polymerizable monomer according to claim 10 or 11, wherein the carbon atoms constituting the main chain of the hydrocarbon group may be substituted by a heteroatom, preferably wherein the heteroatom is an oxygen atom, a nitrogen atom, a sulfur atom, or a silicon atom.

13. A curable composition, comprising:
the polymerizable monomer of any one of claims 1 to 9; and
a polymerization initiator.

14. A resin member, comprising a cured product obtained by using a composition containing the polymerizable monomer of any one of claims 1 to 9.

## Patentansprüche

1. Polymerisierbares Monomer, das durch die folgende allgemeine Formel (1) dargestellt wird: 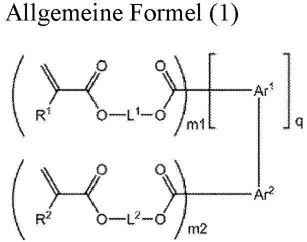 wobei in der allgemeinen Formel (1) Ar¹ und Ar² jeweils eine substituierte oder unsubstituierte aromatische Gruppe darstellen, die eine Valenz aufweist, die aus Divalenz oder Trivalenz ausgewählt ist, und identisch oder verschieden voneinander sein können, L¹ und L² jeweils eine divalente substituierte Kohlenwasserstoffgruppe darstellen, die eine Hauptkette mit einer Anzahl von Atomen in einem Bereich von 2 bis 10 aufweist und mindestens einer Hydroxygruppe enthält, und identisch oder verschieden voneinander sein können, R¹ und R² jeweils Wasserstoff oder eine Methylgruppe darstellen, q 1 ist und m1 und m2 jeweils 1 oder 2 darstellen; vorausgesetzt, dass das polymerisierbare Monomer nicht Folgendes ist:

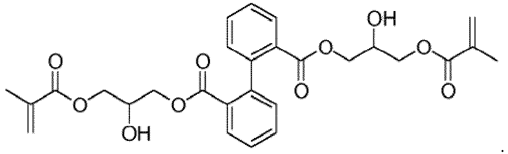

2. Polymerisierbares Monomer nach Anspruch 1, wobei ein Substituent der substituierten aromatischen Gruppe aus einer Gruppe ausgewählt ist, die aus Folgenden besteht: einer monovalenten Kohlenwasserstoffgruppe, die 1 bis 20 Kohlenstoffatome aufweist, -COOR³, -OR³, einer Halogengruppe, einer Aminogruppe, einer Nitrogruppe und einer Carboxylgruppe, und R³ eine monovalente Kohlenwasserstoffgruppe ist, die 1 bis 20 Kohlenstoffatome aufweist.

3. Polymerisierbares Monomer nach Anspruch 1 oder 2, wobei die Kohlenstoffatome, die die Hauptkette der Kohlenwasserstoffgruppe bilden, durch ein Heteroatom substituiert sein können, bevorzugt wobei das Heteroatom ein Sauerstoffatom, ein Stickstoffatom, ein Schwefelatom oder ein Siliciumatom ist.

4. Polymerisierbares Monomer nach einem der Ansprüche 1 bis 3, wobei die Atome, die die Hauptkette der Kohlenwasserstoffgruppe bilden, daran gebunden einen Substituenten aufweisen können, bevorzugt wobei der Substituent eine Alkylgruppe, die 1 bis 3 Kohlenstoffatome aufweist, ein Halogen, -COOR⁴ oder -OR⁴ ist, wobei R⁴ gleich der Alkylgruppe ist, die 1 bis 3 Kohlenstoffatome aufweist.

5. Polymerisierbares Monomer nach einem der Ansprüche 1 bis 4, wobei das polymerisierbare Monomer durch die folgende allgemeine Formel (2) dargestellt wird: 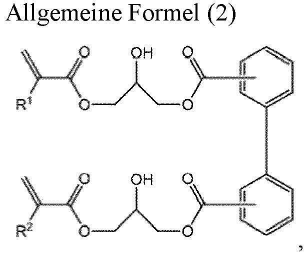 wobei in der allgemeinen Formel (2) R¹ und R² gleich denen sind, die in der allgemeinen Formel (1) gezeigt sind.

6. Polymerisierbares Monomer nach einem der Ansprüche 1 bis 4, wobei das polymerisierbare Monomer durch die folgende allgemeine Formel (3) dargestellt wird: 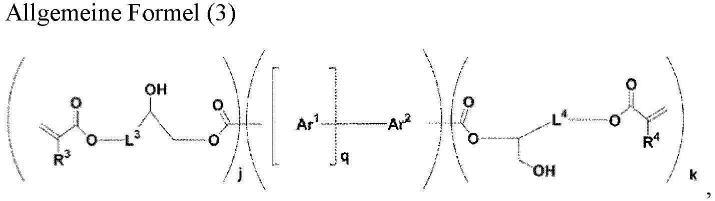 wobei in der allgemeinen Formel (3) Ar¹ und Ar² gleich denen sind, die in der allgemeinen Formel (1) gezeigt sind, außer, dass die Valenz nur Divalenz sein könnte, q gleich dem ist, das in der allgemeinen Formel (1) gezeigt ist, L³ und L⁴ jeweils eine divalente Kohlenwasserstoffgruppe darstellen, die eine Hauptkette mit einer Anzahl von Atomen in einem Bereich von 1 bis 8 aufweist, und identisch oder verschieden voneinander sein können, R³ und R⁴ jeweils Wasserstoff oder eine Methylgruppe darstellen, j 0, 1 oder 2 darstellt, k 0, 1 oder 2 darstellt und j + k = 2.

7. Polymerisierbares Monomer nach Anspruch 6, wobei eine Kombination (j, k) von Werten j und k, die in der allgemeinen Formel (3) gezeigt sind, eine Kombination umfasst, die aus der Gruppe ausgewählt ist, die aus (1, 1) und (0, 2) besteht.

8. Polymerisierbares Monomer nach Anspruch 6, wobei das polymerisierbare Monomer zwei oder mehr Arten von Strukturisomeren umfasst, in denen jeweils eine Kombination (j, k) von Werten j und k, die in der allgemeinen Formel (3) gezeigt sind, aus der Gruppe ausgewählt ist, die aus (2, 0), (1, 1) und (0, 2) besteht.

9. Polymerisierbares Monomer nach Anspruch 8, wobei ein durchschnittlicher Wert der Werte k in allen Molekülen des polymerisierbaren Monomers 0,05 oder mehr und weniger als 2,0 beträgt.

10. Verfahren zur Herstellung eines polymerisierbaren Monomers, wobei das polymerisierbare Monomer zwei oder mehr Arten von Strukturisomeren umfasst, die aus der Gruppe ausgewählt sind, die aus Verbindungen besteht, die durch die folgenden allgemeinen Formeln (6) bis (8) dargestellt werden, wobei das Verfahren mindestens einen Reaktionsschritt zur Ermöglichung umfasst, dass eine Verbindung, die durch die folgende allgemeine Formel (4) dargestellt wird, und eine Verbindung, die durch die folgende allgemeine Formel (5) dargestellt wird, miteinander reagieren: 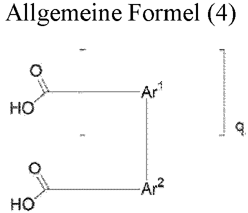 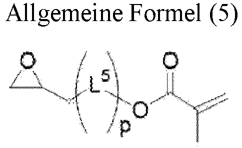 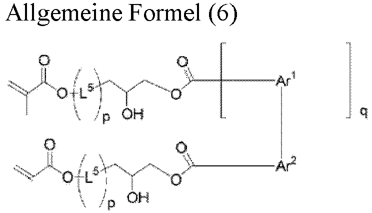 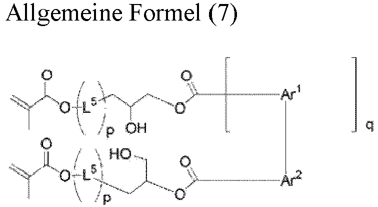 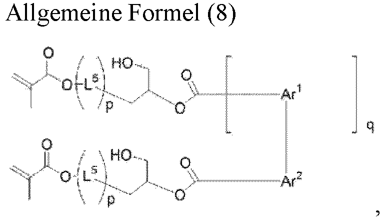 wobei in den allgemeinen Formeln (4) bis (8) Ar¹ und Ar² jeweils eine divalente substituierte oder unsubstituierte aromatische Gruppe darstellen und identisch oder verschieden voneinander sein können, L⁵ eine divalente substituierte oder unsubstituierte Kohlenwasserstoffgruppe darstellt, die eine Hauptkette mit einer Anzahl von Atomen von 1 bis 7 aufweist, p 0 oder 1 darstellt und q 1 ist.

11. Verfahren zur Herstellung eines polymerisierbaren Monomers nach Anspruch 10, wobei ein Substituent der substituierten aromatischen Gruppe aus einer Gruppe ausgewählt ist, die aus Folgenden besteht: einer monovalenten Kohlenwasserstoffgruppe, die 1 bis 20 Kohlenstoffatome aufweist, -COOR³, -OR³, einer Halogengruppe, einer Aminogruppe, einer Nitrogruppe und einer Carboxylgruppe, und R³ eine monovalente Kohlenwasserstoffgruppe ist, die 1 bis 20 Kohlenstoffatome aufweist.

12. Verfahren zur Herstellung eines polymerisierbaren Monomers nach Anspruch 10 oder 11, wobei die Kohlenstoffatome, die die Hauptkette der Kohlenwasserstoffgruppe bilden, durch ein Heteroatom substituiert sein können, bevorzugt wobei das Heteroatom ein Sauerstoffatom, ein Stickstoffatom, ein Schwefelatom oder ein Siliciumatom ist.

13. Härtbare Zusammensetzung, die Folgendes umfasst:
das polymerisierbare Monomer nach einem der Ansprüche 1 bis 9 und
einen Polymerisationsinitiator.

14. Harzelement, das ein gehärtetes Produkt umfasst, das durch Verwendung einer Zusammensetzung, die das polymerisierbare Monomer nach einem der Ansprüche 1 bis 9 enthält, erhalten wird.

## Revendications

1. Monomère polymérisable qui est représenté par la formule générale (1) suivante : 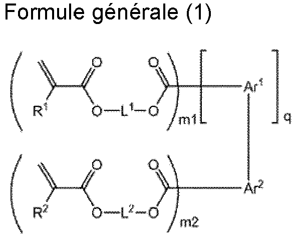 À la formule générale (1), Ar¹ et Ar², qui peuvent être identiques ou différents l'un de l'autre, représentent chacun un groupement aromatique substitué ou non substitué ayant une valence sélectionnée parmi une divalence ou une trivalence, L¹ et L², qui peuvent être identiques ou différents l'un de l'autre, représentent chacun un groupement hydrocarboné divalent substitué comportant une chaîne principale ayant un nombre d'atomes dans la plage de 2 à 10 et contenant au moins un groupement hydroxy, R¹ et R² représentent chacun un hydrogène ou un groupement méthyle, q prend la valeur 1 et m1 et m2 prennent chacun la valeur 1 ou 2 ; à condition que le monomère polymérisable soit autre que le suivant :

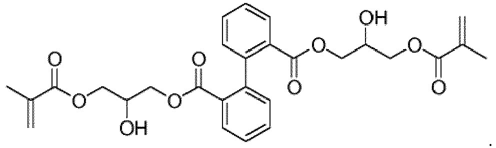

2. Monomère polymérisable selon la revendication 1, où un substituant du groupement aromatique substitué est sélectionné dans un groupe consistant en un groupement hydrocarboné monovalent à 1 à 20 atomes de carbone, -COOR³, -OR³, un groupement halogéné, un groupement amino, un groupement nitro et un groupement carboxyle, et R³ représente un groupement hydrocarboné monovalent à 1 à 20 atomes de carbone.

3. Monomère polymérisable selon la revendication 1 ou 2, où les atomes de carbone constituant la chaîne principale du groupement hydrocarboné peuvent être substitués par un hétéroatome, préférablement où l'hétéroatome est un atome d'oxygène, un atome d'azote, un atome de soufre ou un atome de silicium.

4. Monomère polymérisable selon l'une quelconque des revendications 1 à 3, où les atomes constituant la chaîne principale du groupement hydrocarboné peuvent être liés à un substituant, préférablement où le substituant est un groupement alkyle à 1 à 3 atomes de carbone, un halogène, -COOR⁴ ou -OR⁴, où R⁴ est le même que le groupement alkyle à 1 à 3 atomes de carbone.

5. Monomère polymérisable selon l'une quelconque des revendications 1 à 4, où le monomère polymérisable est représenté par la formule générale (2) suivante : 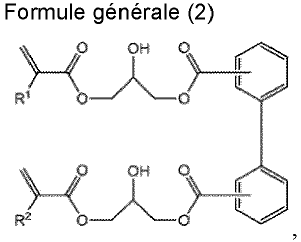 À la formule générale (2), R¹ et R² sont les mêmes que ceux indiqués à la formule générale (1).

6. Monomère polymérisable selon l'une quelconque des revendications 1 à 4, où le monomère polymérisable est représenté par la formule générale (3) suivante : 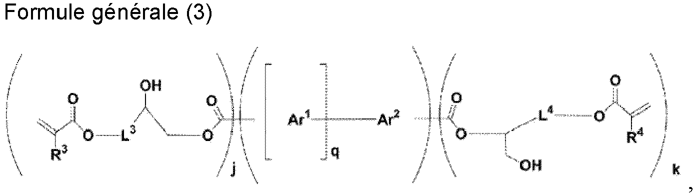 À la formule générale (3), Ar¹ et Ar² sont les mêmes que ceux indiqués à la formule générale (1) à l'exception du fait que la valence peut uniquement être une divalence, q prend la même valeur qu'à la formule générale (1), L³ et L⁴, qui peuvent être identiques ou différents l'un de l'autre, représentent chacun un groupement hydrocarboné divalent comportant une chaîne principale ayant un nombre d'atomes dans la plage de 1 à 8, R³ et R⁴ représentent chacun un hydrogène ou un groupement méthyle, j prend la valeur 0, 1 ou 2, k prend la valeur 0, 1 ou 2 et j+k=2.

7. Monomère polymérisable selon la revendication 6, où une combinaison (j, k) des valeurs j et k indiquées à la formule générale (3) comprend une combinaison sélectionnée dans le groupe consistant en (1, 1) et (0, 2).

8. Monomère polymérisable selon la revendication 6, où le monomère polymérisable comprend deux ou plus types d'isomères structuraux dans chacun desquels une combinaison (j, k) des valeurs j et k indiquées à la formule générale (3) comprend une combinaison sélectionnée dans le groupe consistant en (2, 0), (1, 1) et (0, 2).

9. Monomère polymérisable selon la revendication 8, où une valeur moyenne des valeurs k dans toutes les molécules du monomère polymérisable est égale ou supérieure à 0,05 et inférieure à 2,0.

10. Procédé de production d'un monomère polymérisable, le monomère polymérisable comprenant deux ou plus types d'isomères structuraux sélectionnés dans le groupe consistant en des composés représentés par les formules générales (6) à (8) suivantes, le procédé comprenant au moins une étape de réaction consistant à permettre à un composé représenté par la formule générale (4) suivante et à un composé représenté par la formule générale (5) suivante de réagir l'un avec l'autre : 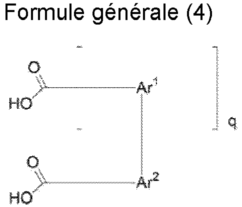 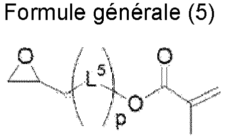 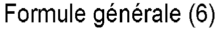 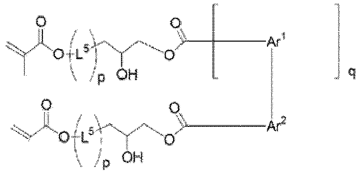 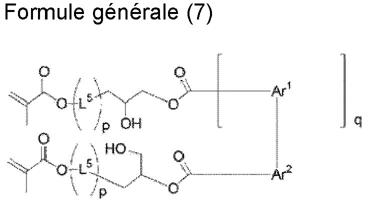 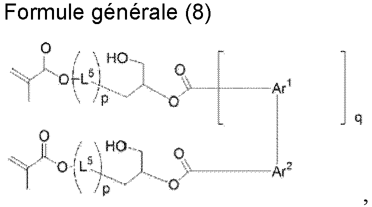 Aux formules générales (4) à (8), Ar¹ et Ar², qui peuvent être identiques ou différents l'un de l'autre, représentent chacun un groupement aromatique divalent substitué ou non substitué, L⁵ représente un groupement hydrocarboné divalent substitué ou non substitué comportant une chaîne principale ayant un nombre d'atomes qui va de 1 à 7, p prend la valeur 0 ou 1 et q prend la valeur 1.

11. Procédé de production d'un monomère polymérisable selon la revendication 10, où un substituant du groupement aromatique substitué est sélectionné dans un groupe consistant en un groupement hydrocarboné monovalent à 1 à 20 atomes de carbone, -COOR³, -OR³, un groupement halogéné, un groupement amino, un groupement nitro et un groupement carboxyle, et R³ représente un groupement hydrocarboné monovalent à 1 à 20 atomes de carbone.

12. Procédé de production d'un monomère polymérisable selon la revendication 10 ou 11, où les atomes de carbone constituant la chaîne principale du groupement hydrocarboné peuvent être substitués par un hétéroatome, préférablement où l'hétéroatome est un atome d'oxygène, un atome d'azote, un atome de soufre ou un atome de silicium.

13. Composition durcissable comprenant :
le monomère polymérisable de l'une quelconque des revendications 1 à 9 ; et un initiateur de polymérisation.

14. Élément en résine comprenant un produit durci obtenu en utilisant une composition qui contient le monomère polymérisable de l'une quelconque des revendications 1 à 9.
